(19) Europäisches Patentamt
European Patent Office
Office européen des brevets

(11) **EP 2 848 915 B1**

(12) **EUROPEAN PATENT SPECIFICATION**

(45) Date of publication and mention
of the grant of the patent:
**20.05.2020 Bulletin 2020/21**

(21) Application number: **14183439.0**

(22) Date of filing: **03.09.2014**

(51) Int Cl.:
*G01N 21/21* (2006.01)          *G01N 21/47* (2006.01)
*G01N 21/55* (2014.01)          *G03G 15/00* (2006.01)
*G01N 21/86* (2006.01)          *B41J 11/00* (2006.01)
*G01N 33/34* (2006.01)

(54) **Sensor device and image forming apparatus incorporating same**

Sensorvorrichtung und Bilderzeugungsvorrichtung damit

Dispositif de capteur et appareil de formation d'images l'intégrant

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.09.2013 JP 2013188965
25.06.2014 JP 2014130734**

(43) Date of publication of application:
**18.03.2015 Bulletin 2015/12**

(73) Proprietor: **Ricoh Company, Ltd.
Tokyo 143-8555 (JP)**

(72) Inventors:
• **Hoshi, Fumikazu**
**Tokyo 143-8555 (JP)**
• **Oba, Yoshihiro**
**Tokyo 143-8555 (JP)**

(74) Representative: **J A Kemp LLP
14 South Square
Gray's Inn
London WC1R 5JJ (GB)**

(56) References cited:
GB-A- 2 148 194        JP-A- H03 240 541
JP-A- 2006 062 842     US-A- 5 075 543
US-A- 5 084 627        US-A- 5 721 434
US-A- 5 915 518        US-A1- 2005 219 305
US-A1- 2006 279 624    US-A1- 2008 063 455
US-A1- 2008 193 157    US-A1- 2013 057 868
US-B1- 6 215 552

**Description**

BACKGROUND

Technical Field

Example embodiments of the present invention generally relate an image forming apparatus incorporating a sensor device.

Background Art

Image forming apparatuses such as digital copying machines and laser printers form an image by transferring a toner image on a recording medium, typified by a printing paper, and heating and pressurizing the recording medium under a specified condition for fixation. When an image is formed, conditions for image formation such as a development condition, a transfer condition, and a fixation condition should be taken into consideration. When a high-quality image is to be formed, in particular, the conditions for image formation need to be set for the type of a recording medium.
This is because the quality of the image is heavily dependent on the recording medium material, thickness, moisture content, smoothness, and coating status. In regard to the smoothness, for example, the stability of a toner becomes low at concave portions of the surface of a printing paper if a proper fixation condition is not chosen. For example, irregularities in color may develop if fixing conditions are not proper for the type of recording medium.
Further, due to the development of image forming apparatuses and the diversification of expression in recent years, hundreds or more types of printing paper have become available as recording media, and a number of brands exist for each of these types for different basis weights and thickness. In order to achieve high-quality image formation, detailed fixation conditions should be set for every one of these brands.
In recent years, the number of brands of paper is increasing for plain paper, coated paper typified by gloss coated paper, matte coated paper, and art paper, plastic sheet, and special paper whose surface is embossed. In recent years, the number of brands of paper is increasing for plain paper, coated paper typified by gloss coated paper, matte coated paper, and art paper, plastic sheet, and special paper whose surface is embossed.
In currently-available image forming apparatuses, a user has to change settings for each tray depending on the brand of paper. For this reason, setting operation could be troublesome. Further, a user is required to know how to identify the type of paper in order to employ the proper settings, and an error in the setting operation leads to an undesired image developed on paper. When the brand of paper is unknown, it is difficult to choose appropriate settings.
For example, JP-3730210-B1 (JP-2004-179721-A) discloses a surface property discriminating device for identifying surface properties of a recording material, including a sensor provided for a recording material conveying system of an image forming apparatus to scan the recording material by relative movement between the recording material and said probe.
JP-2012-127937-A discloses a method for optically scanning the surface of paper placed in a tray and identifying the type of the paper.
JP-2012-127937-A discloses an optical device for identifying the brand of recording paper by irradiating the recording paper with linearly polarized light and detecting the regular reflection light or a polarized component that is orthogonal to the polarized light of the irradiation light included in the diffuse reflection light.
However, it is difficult for conventional devices to easily and precisely identify the type of object.
US 2013/057868 A1, JP 2006-062842 A, US 2006/279624 A1, US 2009/06345 A1, GB 2 148 194 A, JP H03-240541 A, US-5084627 A, US-2008/0063455 A1, US 5 721 434 A, US 6 215 552 B1, US 5 075 543 A, US 2008/193157 A1, US 2005/219305 A1 disclose image forming apparatuses comprising integrated paper characteristic sensing devices. US 5 915 518 A discloses a sensor device for the detection of counterfeit objects, for instance counterfeit of banknotes.

SUMMARY

Embodiments of the present invention described herein provide an image forming apparatus incorporating a sensor device as defined by the appended claims.

BRIEF DESCRIPTION OF THE DRAWINGS

A more complete appreciation of exemplary embodiments and the many attendant advantages thereof will be readily obtained as the same becomes better understood by reference to the following detailed description when considered in connection with the accompanying drawings.

FIG. 1 is a schematic diagram the configuration of a color printer according to an example embodiment of the present invention.

FIG. 2 is an external view of a sensor device according to an example embodiment of the present invention.

FIG. 3 illustrates the configuration of a sensor device according to an example embodiment of the present invention.

FIGS. 4A to 4C illustrate a housing according to an example embodiment of the present invention.

FIG. 5 illustrates the inner structure of a housing according to an example embodiment of the present invention.

FIGS. 6A to 6C illustrate a supporting member according to an example embodiment of the present invention.

FIG. 7 illustrates spring members according to an example embodiment of the present invention.

FIG. 8 illustrates the relative positions of an optical system, a supporting member, and recording paper when the recording paper is inserted into a slit, according to an example embodiment of the present invention.

FIG. 9 illustrates the configuration of an optical system according to an example embodiment of the present invention.

FIG. 10 illustrates the relative positions of an optical system and a slit according to an example embodiment of the present invention.

FIG. 11 illustrates a surface emitting laser array according to an example embodiment of the present invention.

FIG. 12 illustrates the angle of incidence of irradiation light on recording paper, according to an example embodiment of the present invention.

FIG. 13 illustrates the relative positions of a first photosensor and a second photosensor according to an example embodiment of the present invention.

FIG. 14 illustrates the arrangement of a third photosensor according to an example embodiment of the present invention.

FIG. 15A illustrates surface regular reflection light.

FIG. 15B illustrates surface diffuse reflection light.

FIG. 15C illustrates internal reflection light.

FIG. 16 illustrates the light that enters a polarizing filter, according to an example embodiment of the present invention.

FIG. 17 illustrates the light received by the second photosensor, according to an example embodiment of the present invention.

FIG. 18 illustrates the light received by the second and third photosensors, according to an example embodiment of the present invention.

FIG. 19 illustrates the configuration of a processing device according to an example embodiment of the present invention.

FIGS. 20A to 20C illustrate how a supporting member moves when recording paper is inserted into a slit, according to an example embodiment of the present invention.

FIG. 21 illustrates the pressing force exerted by a plurality of spring members on a supporting member, according to an example embodiment of the present invention.

FIG. 22 illustrates the pulling force applied to recording paper when the recording paper is inserted into a slit, according to an example embodiment of the present invention.

FIG. 23 illustrates brand-specific output level data according to an example embodiment of the present invention.

FIG. 24 is a flowchart of the processes executed by a CPU of a processing device when brand identification processes are performed, according to an example embodiment of the present invention.

FIGS. 25A and 25B illustrate the relative movement of recording paper according to an example embodiment of the present invention.

FIG. 26 illustrates a plurality of detection positions according to an example embodiment of the present invention.

FIG. 27 illustrates a first modification (1) of a sensor device according to an example embodiment of the present invention.

FIG. 28 illustrates a first modification (2) of a sensor device according to an example embodiment of the present invention.

FIG. 29 illustrates the characteristics of transmission light according to an example embodiment of the present invention.

FIG. 30 illustrates brand-specific output level data that corresponds to the first modification of a sensor device, according to an example embodiment of the present invention.

FIG. 31 illustrates a second modification of a sensor device according to an example embodiment of the present invention.

FIG. 32 illustrates a third modification (1) of a sensor device according to an example embodiment of the present invention.

FIG. 33 illustrates a third modification (2) of a sensor device according to an example embodiment of the present invention.

FIG. 34 illustrates brand-specific output level data that corresponds to the third modification of a sensor device, according to an example embodiment of the present invention.

FIG. 35 illustrates a sensor for detecting the insertion of recording paper, according to an example embodiment of the present invention.

FIGS. 36A and 36B illustrate the operation of a sensor for detecting the insertion of recording paper, according to an example embodiment of the present invention.

FIG. 37 illustrates a modification of a surface emitting laser array according to an example embodiment of the present invention.

FIG. 38 illustrates a fourth modification of a sensor device according to an example embodiment of the present invention.

FIG. 39 illustrates a fifth modification (1) of a sensor device according to an example embodiment of the present invention.

FIG. 40 illustrates a fifth modification (2) of a sensor device according to an example embodiment of the present invention.

FIG. 41 illustrates a sixth modification (1) of a sensor device according to an example embodiment of the present invention.

FIG. 42 illustrates a sixth modification (2) of a sensor device according to an example embodiment of the present invention.

FIG. 43 illustrates a seventh modification (1) of a sensor device according to an example embodiment of the present invention.

FIG. 44 illustrates a seventh modification (2) of a sensor device according to an example embodiment of the present invention.

FIG. 45 illustrates an irradiation area according to an example embodiment of the present invention.

FIG. 46 illustrates a desired shape of an opening according to an example embodiment of the present invention.

FIG. 47 illustrates a first modification of an opening according to an example embodiment of the present invention.

FIG. 48 illustrates the arrangement of a light source and a plurality of photosensors with reference to an opening of a modification, according to an example embodiment of the present invention.

FIG. 49A illustrates a second modification of an opening according to an example embodiment of the present invention.

FIG. 49B illustrates a third modification of an opening according to an example embodiment of the present invention.

FIG. 50A illustrates a fourth modification of an opening according to an example embodiment of the present invention.

FIG. 50B illustrates a fifth modification of an opening according to an example embodiment of the present invention.

FIGS. 51A and 51B illustrate other methods of preventing recording paper from being caught at an edge of an opening, according to an example embodiment of the present invention.

The accompanying drawings are intended to depict exemplary embodiments of the present disclosure and should not be interpreted to limit the scope thereof. The accompanying drawings are not to be considered as drawn to scale unless explicitly noted.

DETAILED DESCRIPTION

The terminology used herein is for the purpose of describing particular embodiments only and is not intended to be limiting of the present invention. As used herein, the singular forms "a", "an" and "the" are intended to include the plural forms as well, unless the context clearly indicates otherwise. It will be further understood that the terms "includes" and/or "including", when used in this specification, specify the presence of stated features, integers, steps, operations, elements, and/or components, but do not preclude the presence or addition of one or more other features, integers, steps, operations, elements, components, and/or groups thereof.

In describing example embodiments shown in the drawings, specific terminology is employed for the sake of clarity. However, the present disclosure is not intended to be limited to the specific terminology so selected and it is to be understood that each specific element includes all technical equivalents that have the same structure, operate in a similar manner, and achieve a similar result.

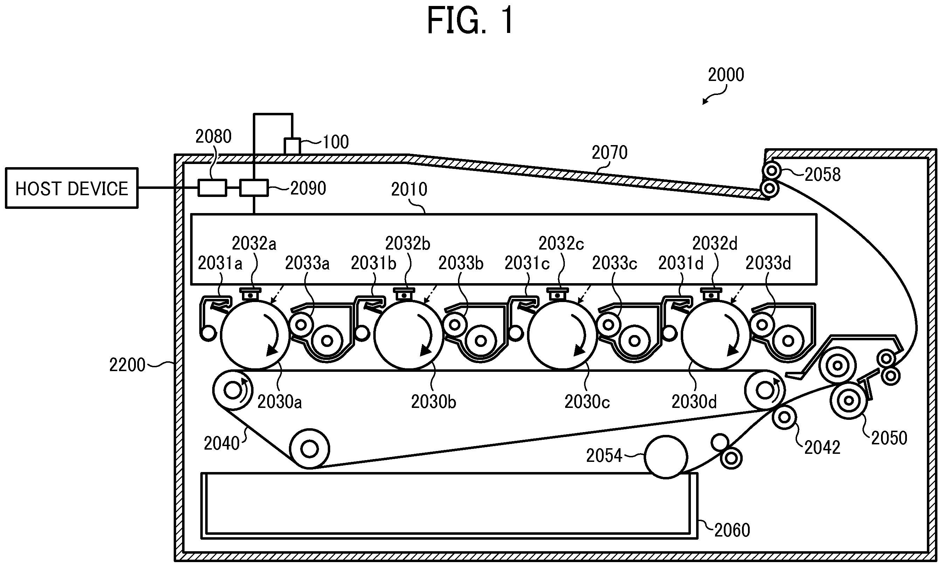

Some embodiments of the present invention are described with reference to FIGS. 1 to 26.

FIG. 1 is a schematic diagram illustrating the configuration of a color printer 2000 according to an example embodiment of the present invention.

The color printer 2000 according to the present example embodiment is a tandem-type multicolor printer that forms a full-color image by superimposing multiple images of four colors (black, cyan, magenta, and yellow) on top of one another. The color printer 2000 includes a sensor device 100, an optical scanner 2010, four photoreceptor drums (2030a, 2030b, 2030c, 2030d), four cleaning units (2031a, 2031b, 2031c, 2031d), four charging devices (2032a, 2032b, 2032c, 2032d), four development rollers (2033a, 2033b, 2033c, 2033d), a transfer belt 2040, a transfer roller 2042, a fixing device 2050, a paper feed roller 2054, an ejection roller 2058, a paper feed tray 2060, a paper output tray 2070, a communication controller 2080, an optical sensor 2245, a printer controller 2090, an operation panel, and printer housing 2200.

The communication controller 2080 controls bidirectional communication with a host device (for example, a personal computer (PC)) through a network or the like.

The printer controller 2090 includes, for example, a central processing unit (CPU), a read-only memory (ROM) in which a program described by CPU-readable codes and various kinds of data used for executing the program are stored, a random access memory (RAM) that serves as a working memory, an amplifier circuit, and an analog-to-digital (A/D) converter that converts an analog signal to a digital signal. Further, the printer controller 2090 controls elements under instructions from a host device, and transfers the image data sent from the host device to the optical scanner 2010. Note that optimal developing conditions and transferring conditions for several brands of recording paper, which can be properly handled as a recording medium by the color printer 2000, are stored in the ROM on a brand-by-brand basis as a "development and transfer table".

The operation panel has a plurality of keys used by an operator to perform several kinds of settings or processes, and a display unit that displays several kinds of information.

The photoreceptor drum 2030a, the charging device 2032a, the development roller 2033a, and the cleaning unit 2031a are used as a unit, and together configure an image forming station that forms a black image. Hereinafter, this image forming station may be referred to as a K-station.

The photoreceptor drum 2030b, the charging device 2032b, the development roller 2033b, and the cleaning unit 2031b are used as a unit, and together configure an image forming station that forms a cyan image. Hereinafter, this image forming station may be referred to as a C-station.

The photoreceptor drum 2030c, the charging device 2032c, the development roller 2033c, and the cleaning unit 2031c are used as a unit, and together configure an image forming station that forms a magenta image. Hereinafter, this image forming station may be referred to as an M-station.

The photoreceptor drum 2030d, the charging device 2032d, the development roller 2033d, and the cleaning unit 2031d are used as a unit, and together configure an image forming station that forms a yellow image. Hereinafter, this image forming station may be referred to as a Y-station.

A photosensitive layer is formed on the surface of each of the photoreceptor drums. The photoreceptor drums rotate in the direction of the arrows as illustrated in FIG. 1.

Each of the charging devices evenly charges the surface of the associated photoreceptor drum.

The optical scanner 2010 scans each of the surfaces of the electrically-charged photoreceptor drums, with the light that is modulated for each color based on the color image data (i.e., black image data, cyan image data, magenta image data, and yellow image data) received from the printer controller 2090. Note that the surface of each of the photoreceptor drums is scanned, Accordingly, a latent image that corresponds to the image data of each color is formed on the surface of each of the photoreceptor drums. Here, each of the photoreceptor drums is an image bearer that carries an image. Each of the latent images moves towards the corresponding development roller as the photoreceptor drum rotates.

A toner form the corresponding toner cartridge is thinly and evenly applied to the surface of each of the development rollers as it rotates. Then, the toner applied to the surface of each of the development rollers moves and adheres to the portions of the surface of the corresponding photoreceptor drum that are irradiated with light by the optical scanner 2010. In other words, the development roller renders a latent image manifest by making the toner adhere to the latent image formed on the surface of the corresponding photoreceptor drum. Each of the toner images moves towards the transfer belt 2040 as the photoreceptor drum rotates.

Each of the toner images of yellow, magenta, cyan, and black is sequentially transferred to the transfer belt 2040 with specified timing. Then, the transferred toner images are superimposed on top of one another to form a color image.

The paper feed tray 2060 stores recording papers therein. The paper feed roller 2054 is arranged near the paper feed tray 2060, and the paper feed roller 2054 takes a piece of recording paper from the paper feed tray 2060. Then, the recording paper is fed between the transfer belt 2040 and the transfer roller 2042 with specified timing. Accordingly, the toner image on the transfer belt 2040 is transferred to the recording paper. The recording paper on which the toner image has been transferred is conveyed to the fixing device 2050.

At the fixing device 2050, heat and pressure are applied to the recording paper to fix the toner on the recording paper. The recording paper on which the toner is fixed is conveyed to the paper output tray 2070 through the ejection roller 2058.

Each of the cleaning units 2031 a, 2031b, 2031c, and 2031 d removes the residual toner left on the surface of the corresponding photoreceptor drum. The surface of the photoreceptor drum from which the residual toner has been removed moves back to a position where the surface of the photoreceptor drum faces the corresponding charging device.

The sensor device 100 is detached from the printer housing 2200, and is disposed near the operation panel such that an operator can pick it up with his/her hand. The sensor device 100 is used to identify the brand of the recording paper.

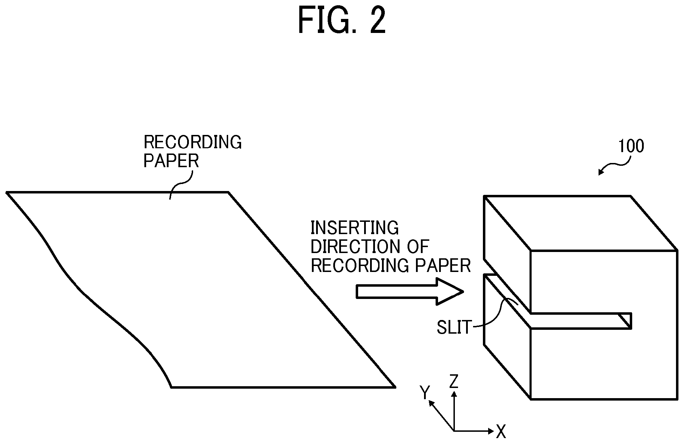

FIG. 2 is an external view of the sensor device 100 according to an example embodiment of the present invention. As illustrated in FIG. 2 as an example, the sensor device 100 is shaped like a quadrangular prism, and has a slit in the insertion direction of recording paper. In the XYZ three-dimensional orthogonal coordinate system according to the present example embodiment, it is assumed that the direction orthogonal to the surface of the recording paper is a Z-axis direction, and that the direction in which the recording paper is inserted into the slit is the +X direction.

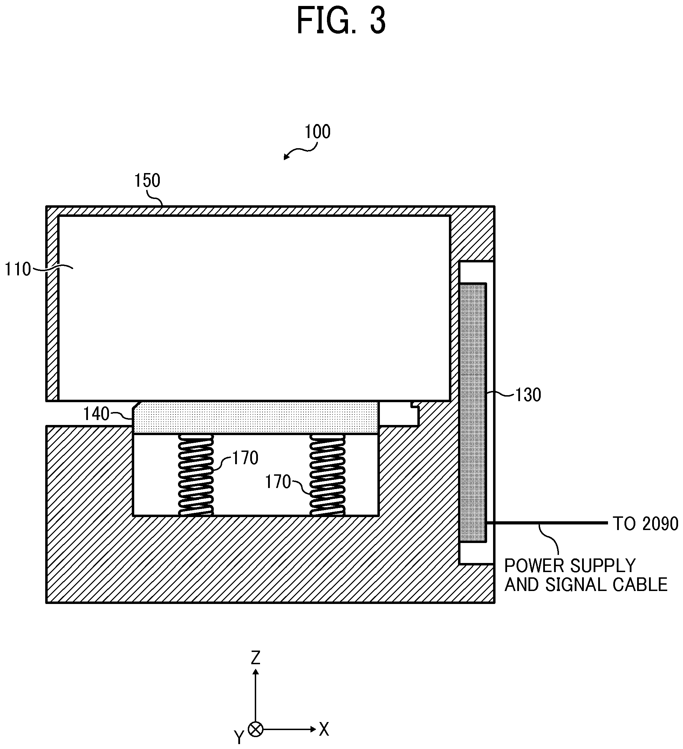

FIG. 3 illustrates the configuration of a sensor device 100 according to an example embodiment of the present invention. As illustrated in FIG. 3 as an example, the sensor device 100 includes an optical system 110, a processing device 130, a supporting member 140, a housing 150, and a plurality of spring members 170. In FIG. 3, the wall on the -Y side of the housing 150 is removed for ease of illustration of the inner structure.

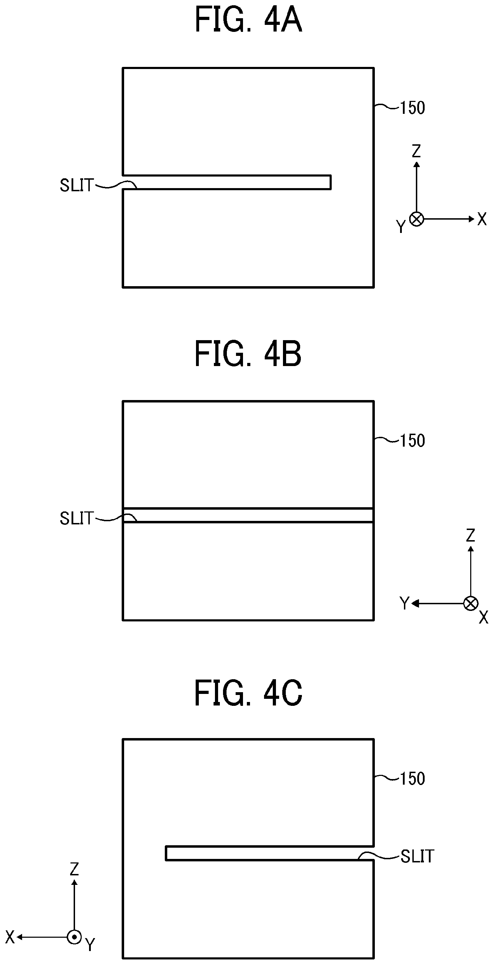

FIGS. 4A to 4C illustrate the housing 150 according to an example embodiment of the present invention. As illustrated in FIGS. 4A to 4C, a slit is formed on the -X-side surface, and the slit penetrates to the +Y-side surface and the -Y-side surface. In other words, the housing 150 has a slit of prescribed length in the direction in which the recording paper is inserted.

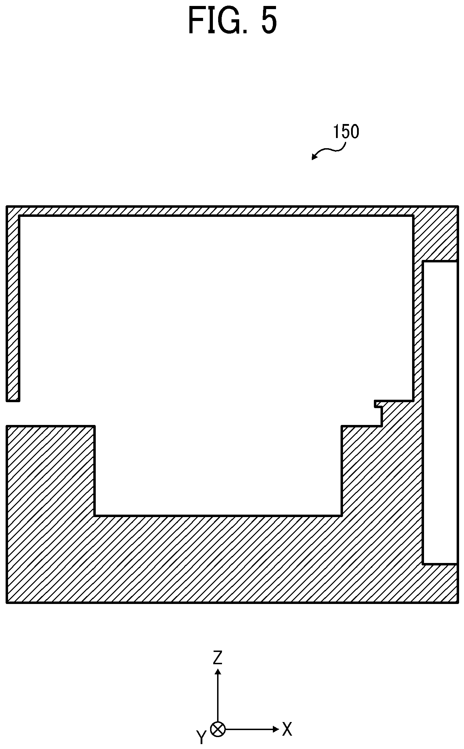

FIG. 5 illustrates the XZ cross section of the housing 150 according to an example embodiment of the present invention. As illustrated in FIG. 5, the housing 150 has inner space in which the optical system 110, the processing device 130, the supporting member 140, and the spring members 170 are accommodated.

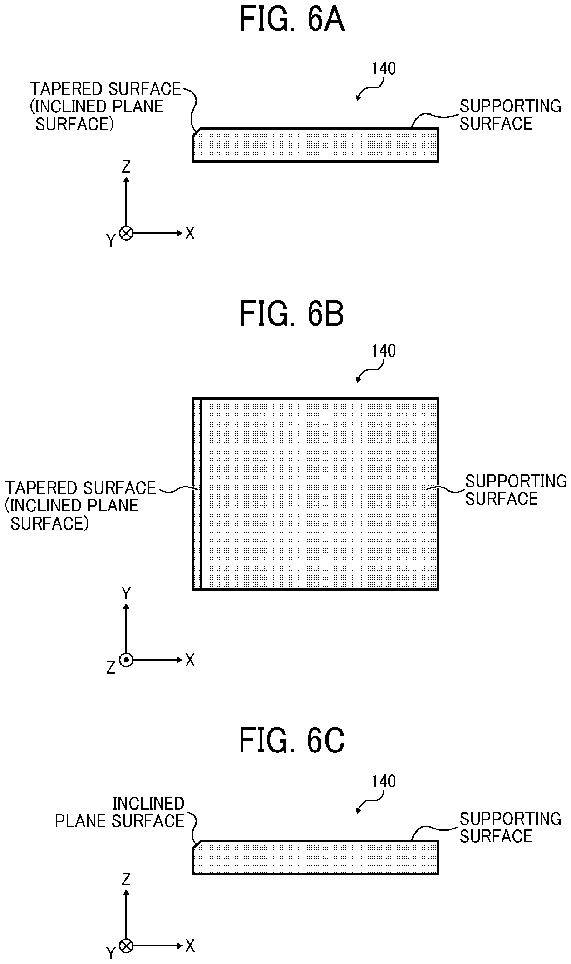

FIGS. 6A to 6C illustrate the supporting member 140 according to an example embodiment of the present invention. As illustrated in FIGS. 6A and 6B, in the present example embodiment, the supporting member 140 has a supporting surface on the +Z side parallel to the XY surface, and supports the recording paper by that supporting surface. At an edge on the -X side of the supporting surface, a tapered surface (inclined plane surface) is formed to guide the recording paper to the supporting surface. Alternatively, as illustrated in FIG. 6C, an inclined curved surface may be formed in place of the tapered surface. Such a curved surface has a round surface at an edge on the -X side of the supporting surface.

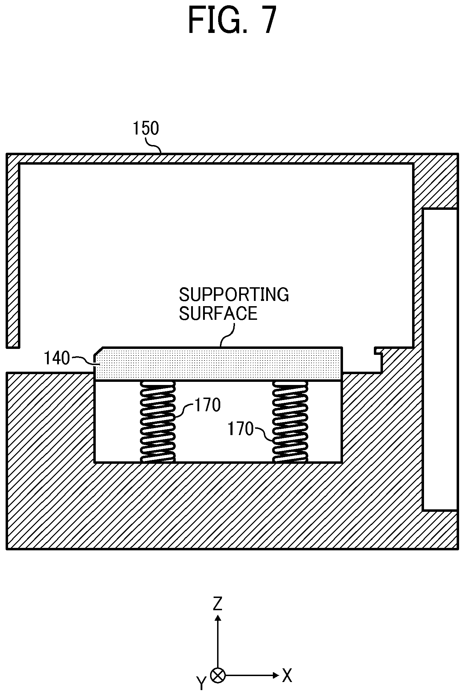

FIG. 7 illustrates the spring members 170 according to an example embodiment of the present invention. As illustrated in FIG. 7, the -Z-side edges of the spring members 170 are fixed to the housing 150, and the +Z-side edges of the spring members 170 are fixed to the -Z side surface of the supporting member 140. In other words, the supporting member 140 is attached to the housing 150 through the spring members 170.

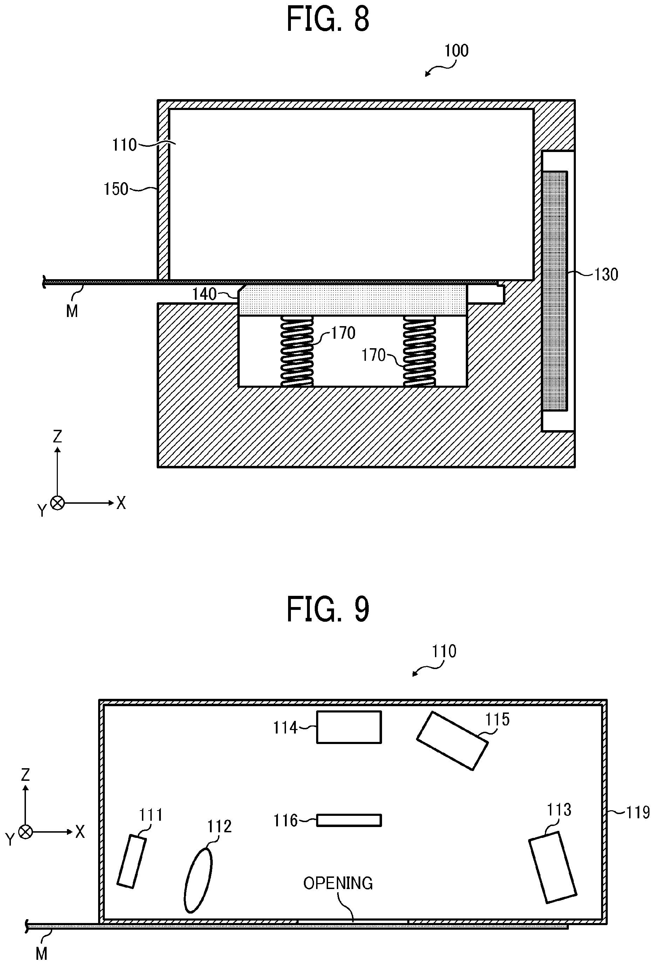

FIG. 8 illustrates the relative positions of the optical system 110, the supporting member 140, and recording paper M when the recording paper M is inserted into the slit, according to an example embodiment of the present invention. As illustrated in FIG. 3, the optical system 110 is disposed on the +Z-side of the supporting member 140. As illustrated in FIG. 8, when the recording paper M is inserted into the slit, the supporting member 140 is pressed down by the recording paper M, and the recording paper M is fed between the -Z-side of a dark box 119 of the optical system 110 and the supporting surface of the supporting member 140. In other words, the recording paper M that is inserted into the slit is on the -Z side of the optical system 110.

FIG. 9 illustrates the configuration of the optical system 110 according to an example embodiment of the present invention. As illustrated in FIG. 9, in the present example embodiment, the optical system 110 includes a light source 111, a collimate lens 112, three photosensors 113, 114, and 115, a polarizing filter 116, and the dark box 119 that encloses these elements.

The dark box 119 is a box made of metal such as aluminum, and the surface of the dark box 119 is anodized in black in order to reduce the influence of disturbance light and stray light. The dark box 119 is fixed to the housing 150.

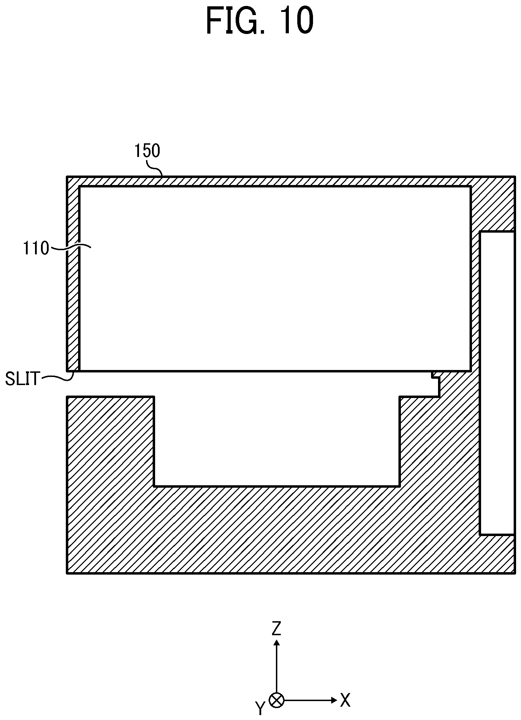

FIG. 10 illustrates the relative positions of the optical system 110 and a slit according to an example embodiment of the present invention. As illustrated in FIG. 10, in the present example embodiment, the position of the -Z-side surface of the dark box 119 is equal to the +Z-side surface of the slit of the housing 150. Note that the position of the -Z-side surface of the dark box 119 in the Z-axis direction is not limited to the above configuration, but may be positioned above the slit of the housing 150.

Before the recording paper M is inserted into the slit, the supporting member 140 is pressed by the spring members 170 towards the +Z-direction, such that the supporting surface of the supporting member 140 softly touches the -Z-side surface of the dark box 119.

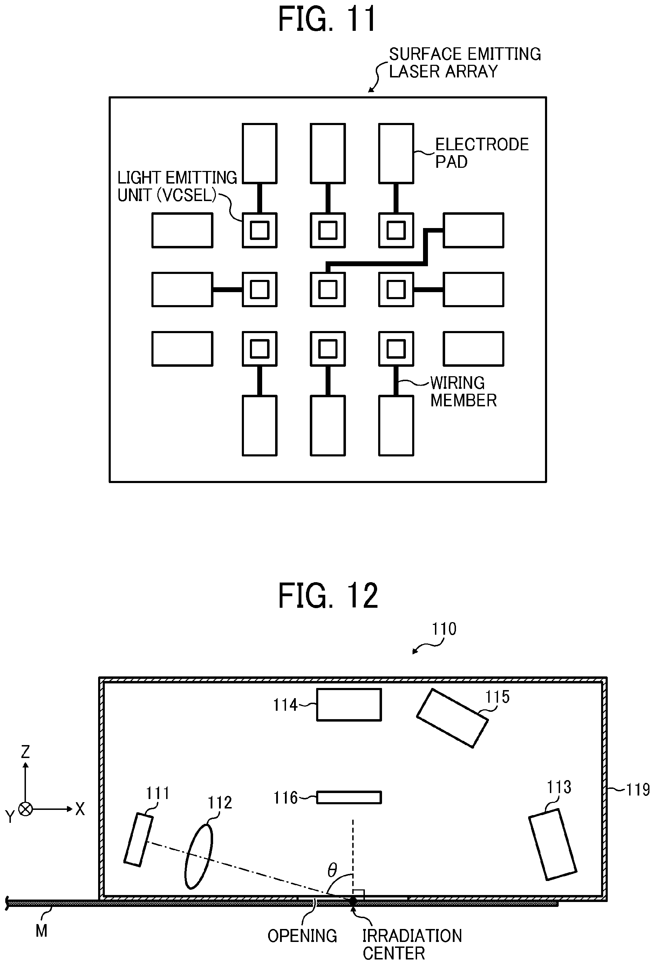

FIG. 11 illustrates a surface emitting laser array according to an example embodiment of the present invention. The light source 111 includes a plurality of light-emitting units. Each of the light-emitting units is a vertical cavity-surface emitting laser (VCSEL). In other words, the light source 111 includes a vertical cavity-surface emitting laser array (VCSEL array). In the present example embodiment described above, as illustrated in FIG. 11, nine light-emitting units are two-dimensionally arranged in the VCSEL array.

The light source 111 is disposed so as to irradiate the recording paper M with the s-polarized linear light. FIG. 12 illustrates the angle of incidence $\theta$ of irradiation light on recording paper, according to an example embodiment of the present invention. In the present example embodiment, the angle of incidence $\theta$ is 80°. The light source 111 is switched on and switched off by the processing device 130.

The collimate lens 112 is disposed on the optical path of the light emitted from the light source 111, and collimates the light. The light that has passed the collimate lens 112 passes through the opening of the dark box 119 to irradiate the recording paper M. Hereinafter, the center of the irradiated area on the surface of the recording paper M is referred to simply as a "irradiation center". Hereinafter, the light that has passed through the collimate lens 112 may be referred to as irradiation light.

Assuming that the light enters the boundary surface of a medium, the plane that includes the incident light beam and the normal line drawn from the point of incidence of the boundary surface is referred to as the incidence plane. When the incident light includes a plurality of light beams, an incidence plane exists for each of the light beams. However, for the purpose of simplification, the incidence plane of the light beam incident on the irradiation center is referred to as the incidence plane of the recording paper M. In other words, the plane that includes the irradiation center and is parallel with the XZ-plane is the incidence plane of the recording paper M.

In the present description, the terms "s-polarized light" and "p-polarized light" are used not only for the incident light on the recording paper M but also for the reflection light. This is for the purpose of simplification, and the linearly polarized light whose polarization direction is the same as that of the incident light (i.e., s-polarized light) on an incidence plane is referred to as the s-polarized light, and the linearly polarized light whose polarization direction is orthogonal to the s-polarized light is referred to as the p-polarized light. These terms are used with reference to the polarization direction of the incident light on the recording paper M.

The polarizing filter 116 is arranged on the +Z-side of the irradiation center. The polarizing filter 116 transmits the p-polarized light and blocks the s-polarized light. Alternatively, the polarizing filter 116 may be replaced with a polarization beam splitter whose capability is equivalent to that of the polarizing filter 116.

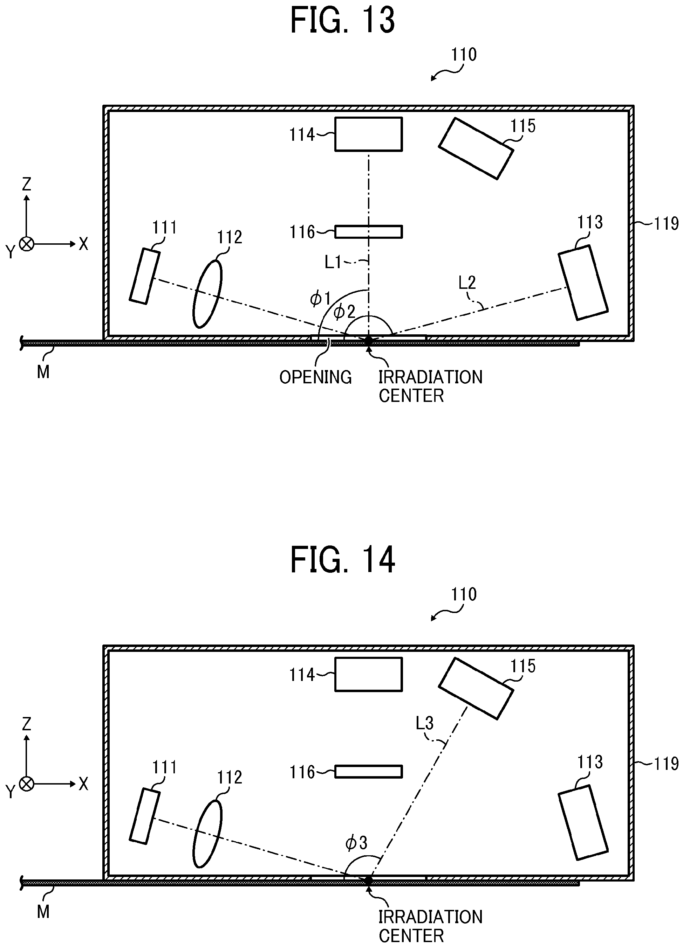

FIG. 13 illustrates the relative positions of the photosensor 113 and the photosensor 114 according to an example embodiment of the present invention. The photosensor 114 is disposed on the +Z-side of the polarizing filter 116, and receives the light that has passed through the polarizing filter 116. As illustrated in FIG. 13, the angle φ1 which the surface of the recording paper M forms with a line L1, which is drawn through the irradiation center, the center of the polarizing filter 116, and the center of the photosensor 114, is 90 degrees.

The photosensor 113 is arranged on +X side of the irradiation center, with reference to the X-axis direction. As illustrated in FIG. 13, the angle φ2 which the surface of the recording paper M forms with a line L2, which is drawn through the irradiation center and the center of the photosensor 113, is 170 degrees.

FIG. 14 illustrates the arrangement of a third photosensor according to an example embodiment of the present invention. The photosensor 115 is arranged between the photosensor 114 and the photosensor 113, with reference to the X-axis direction. As illustrated in FIG. 14, the angle φ3 which the surface of the recording paper M forms with a line L3, which is drawn through the irradiation center and the center of the photosensor 115, is 120 degrees.

Note that the center of the light source 111, the irradiation center, the center of the polarizing filter 116, the centers of the photosensors 113, 114, and 115 are substantially on the same plane.

Note that the direction in which the light source 111 and the photosensors 113, 114, and 115 are arranged is not limited to the above configuration. For example, the direction in which the light source 111 and the photosensors 113, 114, and 115 are arranged may be orthogonal to the direction in which the recording paper is inserted, or may be inclined towards the direction in which the recording paper is inserted.

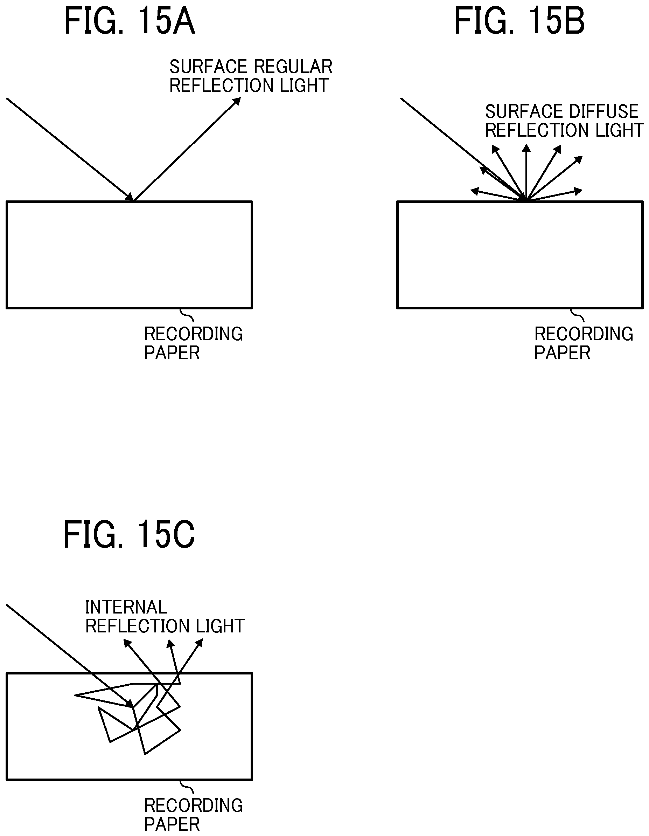

FIG. 15A illustrates surface regular reflection light, and FIG. 15B illustrates surface diffuse reflection light. The light reflected from recording paper when the recording paper is irradiated with light is classified into two kinds of reflected light, consisting of the light reflected at the surface of the recording paper, and the light reflected inside the recording paper. Moreover, the light reflected at the surface of the recording paper is classified into two kinds of reflected light, consisting of the light of regular reflection and the light of diffuse reflection. Hereinafter, the light reflected by regular reflection on the recording paper is referred to as "surface regular reflection light", and the light reflected by diffuse reflection is referred to as "surface diffuse reflection light" for the purpose of simplification, as illustrated in FIGS. 15A and 15B.

The surface of recording paper is composed of plane portions and oblique portions, and the smoothness of the recording paper is determined by the ratio of the plane portions to the oblique portions. The light reflected at a plane portion becomes surface regular reflection light, and the light reflected at an oblique portion becomes surface diffuse reflection light. The surface diffuse reflection light is completely-dispersed reflection light, and it is assumable that the direction of reflection is isotropic. When the smoothness is high, the light quantity of the surface regular reflection light increases accordingly.

FIG. 15C illustrates internal reflection light. When the recording paper is ordinary printing paper, the reflected light from the inside of the recording paper is composed of only diffuse reflection light because multiple scattering occurs inside the fibers of the recording paper. Hereinafter, the reflected light from the inside of the recording paper is referred to as "internal reflection light" for the purpose of simplification, as illustrated in FIG. 15C. The internal reflection light is also completely-dispersed reflection light in a similar manner to the surface diffuse reflection light, and it is assumable that the direction of reflection is isotropic.

The polarization direction of the surface regular reflection light and surface diffuse reflection light towards a photosensor is the same as the polarization direction of the incident light. Note that the polarization direction rotates on the surface of recording paper only when the incident light is reflected on a part of the surface that is inclined in the direction of the rotation with reference to the incident direction. Because the center of the light source, the irradiation center, and the centers of the photosensors are substantially on a same plane, the light whose polarization direction is rotated on the surface of recording paper is not reflected to any of the photosensors.

By contrast, the polarization direction of the internal reflection light is rotated with reference to the polarization direction of the incident light. This happens as the light that enters recording paper is transmitted through fibers and is subject to optical rotation while experiencing multiple scattering, and the polarization direction is rotated.

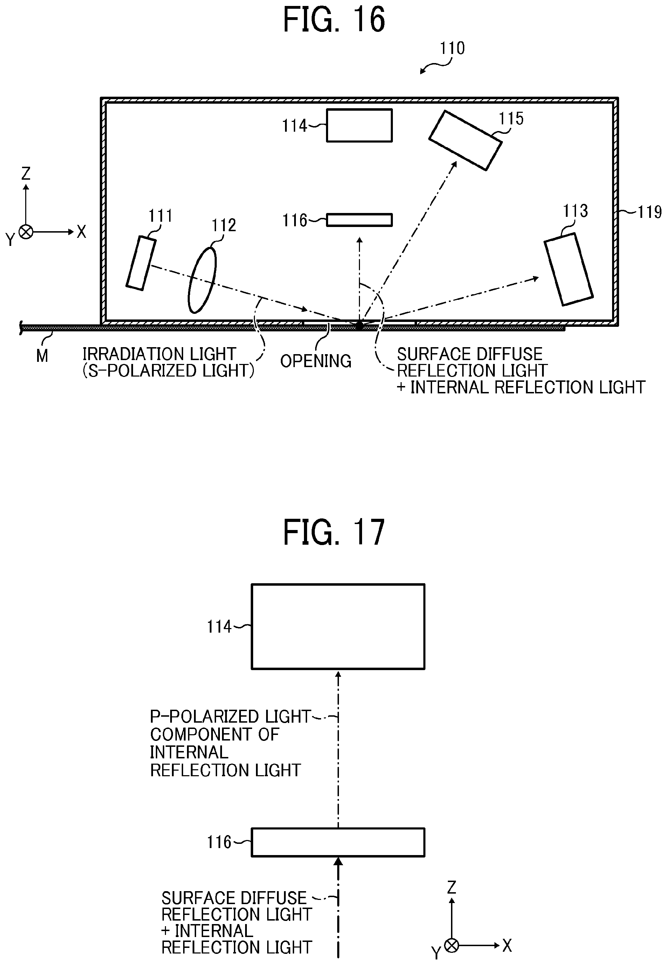

FIG. 16 illustrates the reflected light that enters the polarization filter 116, according to an example embodiment of the present invention. As illustrated in FIG. 16, reflection light including the surface diffuse reflection light and internal reflection light enters the polarizing filter 116.

As the surface diffuse reflection light has the s-polarized light same as that of the incident light, the surface diffuse reflection light that enters the polarizing filter 116 is blocked at the polarizing filter 116. On the other hand, the internal reflection light has both the s-polarized light and p-polarized light, and the p-polarized light passes through the polarizing filter 116. In other words, the p-polarized light included in the internal reflection light is received at the photosensor 114. FIG. 17 illustrates the light received by the photosensor 114, according to an example embodiment of the present invention. Hereinafter, the p-polarized light included in the internal reflection light may be referred to as "p-polarized internal reflection light" for the purpose of simplification. In a similar manner, the s-polarized light included in the internal reflection light may be referred to as "s-polarized internal reflection light".

According to the experiments run by the inventor and his associates, it is known that the amount of the p-polarized internal reflection light correlates with the thickness or density of recording paper. This is because the amount of the p-polarized internal reflection light is dependent upon the length of the path where the light passes through the fibers of the recording paper.

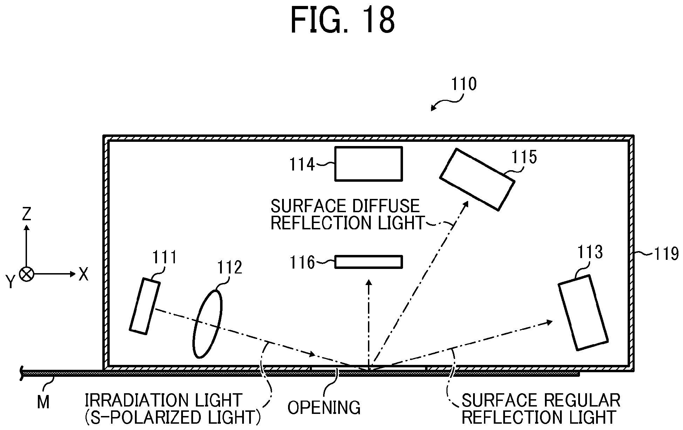

FIG. 18 illustrates the light received by the photosensor 113, according to an example embodiment of the present invention. As illustrated in FIG. 18, reflection light including the surface regular reflection light, surface diffuse reflection light, and internal reflection light enters the photosensor 113. At this light receiving position, the amount of the surface diffuse reflection light and internal reflection light is very small compared with the amount of the surface regular reflection light. For this reason, it can be assumed that the amount of the light received at the photosensor 113 is substantially equivalent to the amount of the surface regular reflection light.

As illustrated in FIG. 18, reflection light including the surface diffuse reflection light and internal reflection light enters the photosensor 115. At this light receiving position, the amount of the internal reflection light is very small compared with the amount of the surface diffuse reflection light. For this reason, it can be assumed that the amount of the light received at the photosensor 115 is substantially equivalent to the amount of the surface diffuse reflection light.

Each of the photosensors transmits an electrical signal to the processing device 130 based on the amount of the received light.

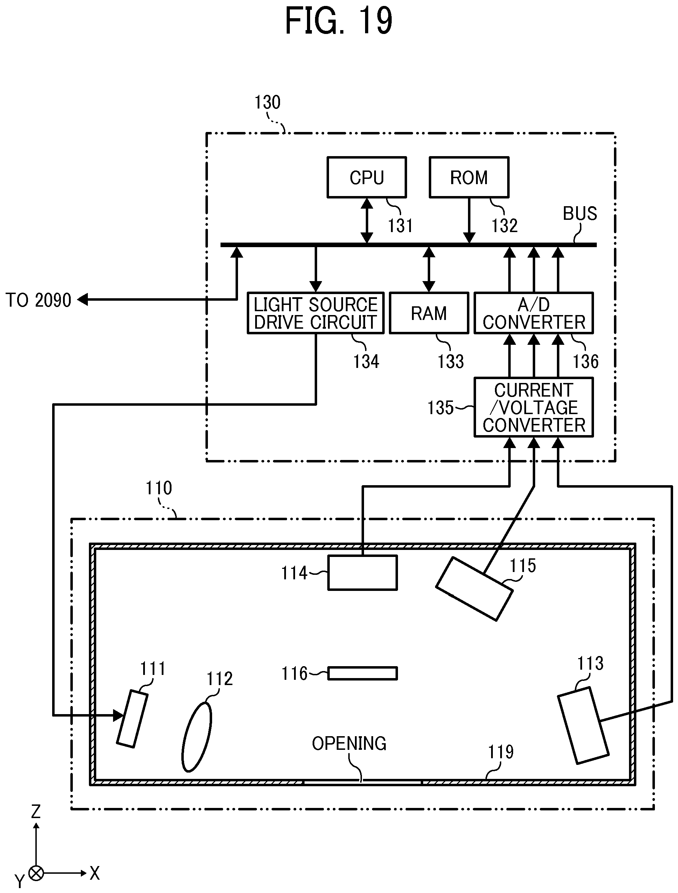

FIG. 19 illustrates the configuration of the processing device 130 according to an example embodiment of the present invention. As illustrated in FIG. 19, the processing device 130 according to the example embodiment of the present invention includes a central processing unit (CPU) 131, a read only memory (ROM) 132, a random access memory (RAM) 133, a light source drive circuit 134, a current/voltage converter 135, and an analog-to-digital (A/D) converter 136. The power is supplied to the processing device 130 from the printer controller 2090 through a cable, and the processing device 130 is controlled by the printer controller 2090 through a wired interface such as a universal serial bus (USB) or RS-232C.

The ROM 132 stores a program described by codes readable by the CPU 131 and various kinds of data used for executing the program. The RAM 133 serves as a working memory.

The light source drive circuit 134 transmits a light-source driving signal to the light source 111 in accordance with the instructions provided by the CPU 131.

The current/voltage converter 135 converts the current signal output from the photosensors into a voltage signal. The A/D converter 136 converts the voltage signal output from the current/voltage converter 135 from an analog signal to a digital signal.

The CPU 131 identifies the brand of recording paper according to the program stored in the ROM 132. The printer controller 2090 is notified of the result of identification.

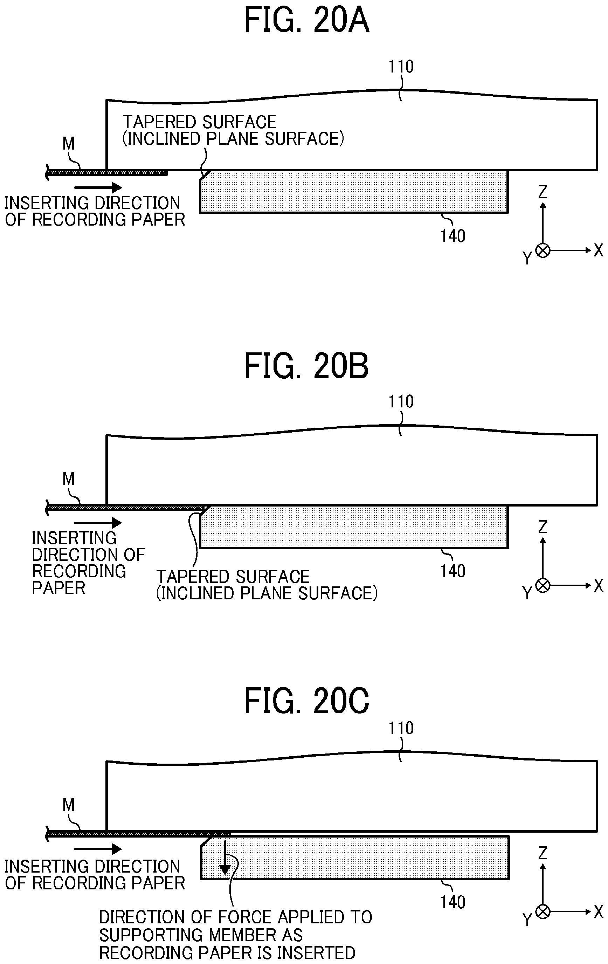

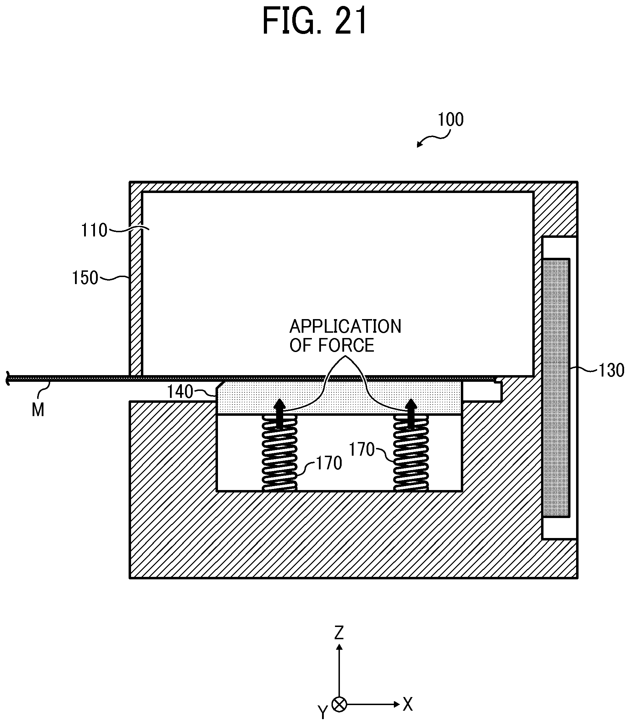

FIGS. 20A to 20C illustrate how the supporting member 140 moves when the recording paper M is inserted into a slit, according to an example embodiment of the present invention. FIG. 21 illustrates the pressing force exerted by the spring members 170 on the supporting member 140, according to an example embodiment of the present invention. When the recording paper M is inserted into the slit and the front end of the recording paper M reaches the tapered surface of the supporting member 140, the supporting member 140 is pressed down towards the -Z side. Then, the spring members 170 contract, and the supporting member 140 moves towards the -Z side by the thickness of the recording paper M. Accordingly, as illustrated in FIG. 21, the supporting member 140 is pressed towards the +Z side due to the resilience force of the spring members 170. Note that a guide is provided for the housing 150 such that the supporting member 140 can smoothly move in the Z-axis direction.

As a result, the recording paper M in full contact with the optical system 110. By so doing, the irradiation position or angle of incidence of the light is fixed with reference to the recording paper M, and a stable amount of reflection light can be obtained. In other words, an improved precision is achieved in identifying the brand of the recording paper M. If the recording paper M is not in full contact with the optical system 110, the irradiation position or angle of incidence of the light fluctuates, and the brand of the recording paper M cannot be identified with precision.

Moreover, when the recording paper M is in full contact with the optical system 110, the optical system 110 becomes completely closed and the noise generated by disturbance light can be prevented. Further, the light (laser light) can be prevented from leaking to the outside of the sensor device 100, and the security for an operator is improved accordingly.

The intensity of the pressing force applied to the supporting member 140 by the spring members 170 is controlled such that an operator can easily pull out the recording paper M from the sensor device 100.

Here, the holding force applied to the recording paper M by the optical system 110 and the supporting member 140 is controlled such that the recording paper M can smoothly move without being caught in the sensor device 100 when the recording paper M is moved relative to the sensor device 100. If the pressing force applied to the recording paper M is too great, it may become difficult to smoothly move the recording paper M, or the recording paper M may be damaged when the recording paper M is moved relative to the sensor device 100. On the other hand, if the pressing force applied to the recording paper M is too weak, a gap is created between the recording paper M and the optical system 110, and the brand of the recording paper M can not be identified with precision.

When very thin recording paper is inserted into a narrow gap, such insertion may fail due to crimping caused to the recording paper. In order to prevent such crimping, a mechanism that increases the gap when recording paper is to be inserted may be provided, but it is likely that such provision leads to complication in structure or an increase in cost.

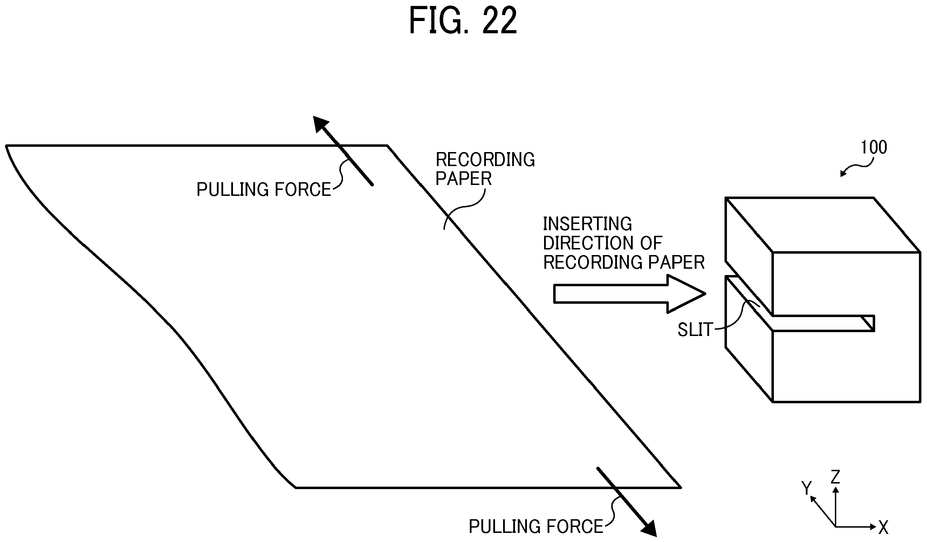

FIG. 22 illustrates the pulling force applied to recording paper when the recording paper is inserted into a slit, according to an example embodiment of the present invention. In the present example embodiment, a slit is provided on the -X-side surface of the housing 150 to which the recording paper is inserted, and the slit penetrates to +Y-side surface and the -Y-side surface that are adjacent to the -X-side surface. Accordingly, in the present example embodiment, as illustrated in FIG. 22, even very thin recording paper can be fed between the optical system 110 and the supporting member 140 without causing bending or crimping, by holding the recording paper with both hands and inserting the recording paper into the slit with tension (pulling force) being applied to the right and left sides of the recording paper with reference to the insertion direction.

In the present example embodiment, a plurality of brands of recording paper, which are compatible with the color printer 2000, are inserted into the slit of sensor device 100, and the output levels of the photosensors when the light source 111 is switched on are obtained. The results of the above obtaining processes are stored in the ROM 1 32 of the processing device 130 as "brand-specific output level data". The brand-specific output level data refers to a database in which the output levels of the photosensors, which are obtained for the already-known different brands of recording paper, are recorded.

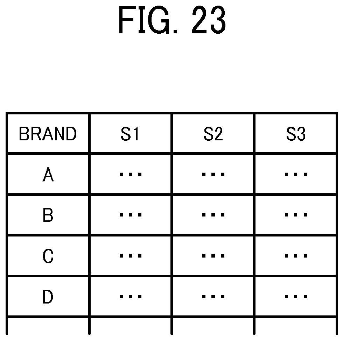

FIG. 23 illustrates brand-specific output level data according to an example embodiment of the present invention. Here, the output levels of the photosensor 113, the photosensor 114, and the photosensor 115 are referred to as S1, S2, and S3, respectively.

Next, processes in which the brand of the recording paper M is identified (brand identification processes) when the brand of the recording paper M is unknown are described.

Firstly, the operation performed by an operator in the brand identification processes are described. 1. The sensor device 100 is placed on the flat surface arranged near the operation panel. 2. To-be-identified recording paper is inserted into the slit of the sensor device 100 with tension (pulling force) being applied to both sides of the recording paper in the Y-axis direction. 3. A request for an identification process is made through the operation panel.

This request for an identification process is sent to the processing device 130 of the sensor device 100 through the operation panel and the printer controller 2090.

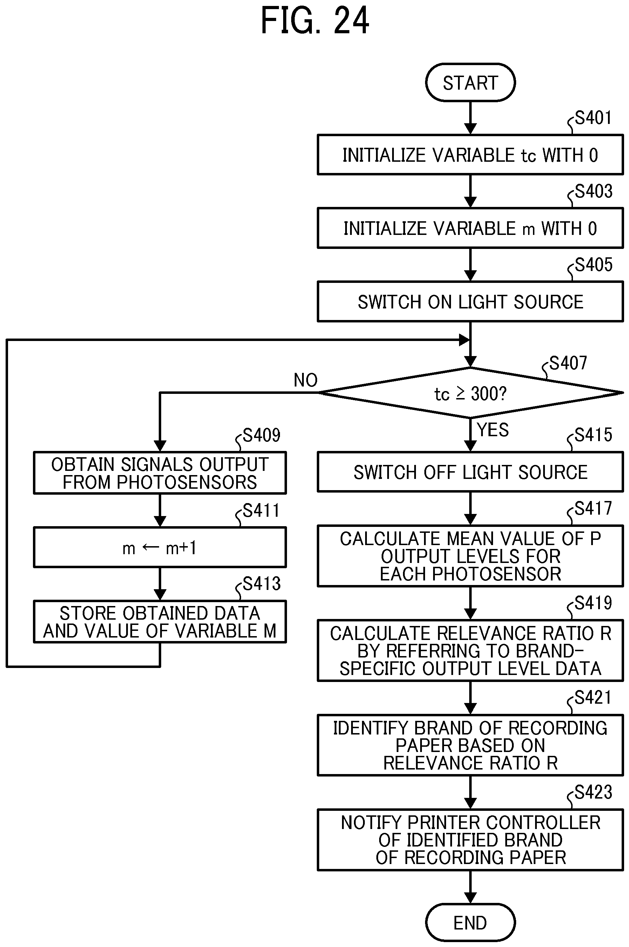

Once the request for an identification process is received, the processing device 130 starts a brand identification process. FIG. 24 is a flowchart of a series of computational algorithms executed by the CPU 131 of the processing device 130 when brand identification processes are performed.

In the first step S401, a variable tc that indicates the value of a time counter is initialized with 0. Here, it is assumed that the variable tc is increased by 1 (+1) for every 10 milliseconds (msec) in the interruption process of a timer.

In the next step S403, a variable m that indicates the number of times the signals output from photosensors have been obtained is initialized with 0.

In step S405, the light source 111 is switched on. Here, a plurality of light-emitting units are switched on at the same time.

In the next step S407, whether or not the variable tc is equal to or greater than "300" is determined. In other words, whether or not three seconds have passed since the light source 111 was switched on is determined. When this determination is negative, the process shifts to step S409.

In step S409, the signals output from photosensors are obtained.

In the next step S411, the variable m is increased by 1 (+1).

In the next step S413, the data obtained in step S409 is stored in the RAM 133 together with the value of the variable m. Then, the process returns to step S407.

The processes in steps S409 to S413 are repeated until the determination in step S407 becomes positive.

When the determination becomes positive in step S407, the process shifts to step S415.

In step S415, the light source 111 is switched off. Assuming that the value of the variable m is "P", P pieces of data are stored in the RAM 133 for each of the photosensors.

In step S417, a mean value of P output levels is calculated for each photosensor. The mean values of the output level of the photosensor 113, the photosensor 114, and the photosensor 115 are referred to as S1', S2', and S3', respectively. S1', S2', and S3' are used as measurement data.

In step S419, the brand-specific output level data stored in the ROM 132 is referred to, and a relevance ratio R is calculated for each brand by using formula (1) below.

[Formula 1]

$$ R = \left(1 - \left|\frac{S1 - S1'}{S1 + S1'}\right|\right) \times \left(1 - \left|\frac{S2 - S2'}{S2 + S2'}\right|\right) \times \left(1 - \left|\frac{S3 - S3'}{S3 + S3'}\right|\right) \quad \cdots\cdots \ (1) $$

In step S421, the brand for which the calculated relevance ratio R becomes the greatest is identified as the brand of the recording paper.

In step S423, the printer controller 2090 is notified of the identified brand of the recording paper. Then, the brand identification process is terminated.

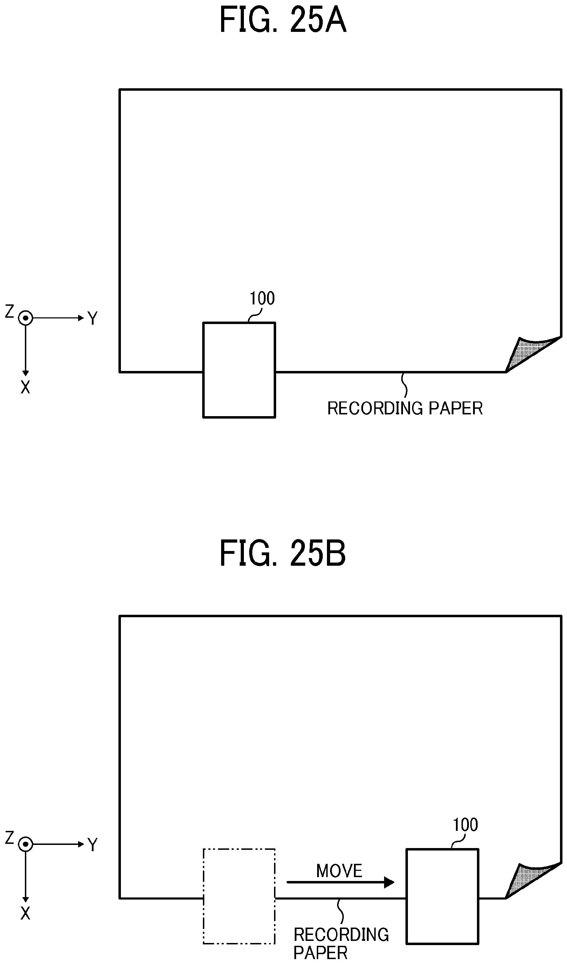

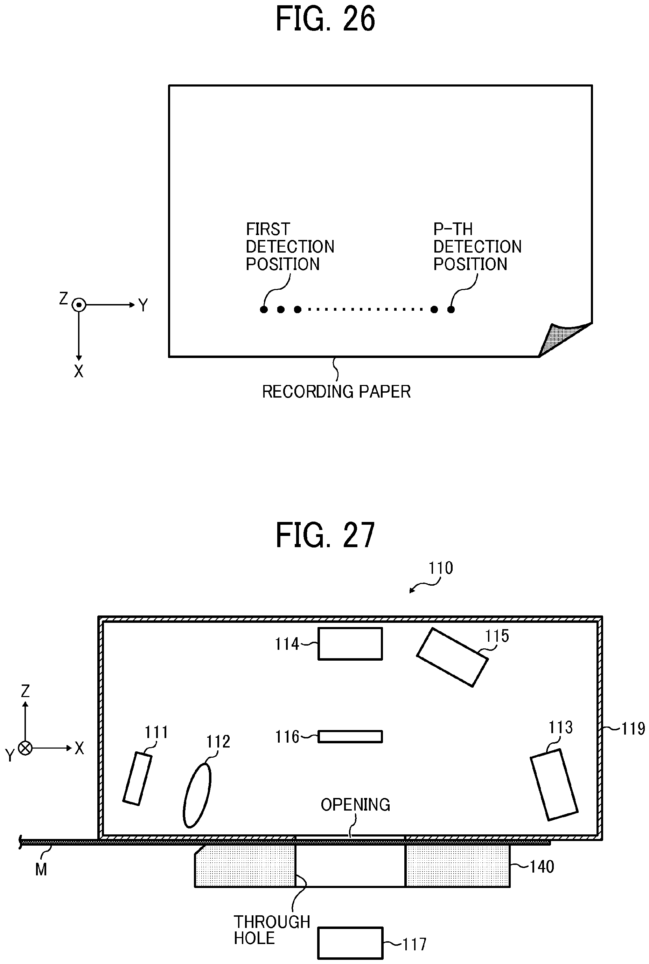

FIGS. 25A and 25B illustrate the relative movement of recording paper according to an example embodiment of the present invention. FIG. 26 illustrates a plurality of detection positions according to an example embodiment of the present invention. An operator may move at least one of the sensor device 100 and the recording paper M, as illustrated in FIGS. 25A and 25B, while the light source 111 is being switched on in the brand identification processes. In such cases, different P positions on the recording paper M are used as detection positions, as illustrated in FIG. 26. Accordingly, nonuniformity in the state of surface or the density of the fiber of the recording paper is averaged, and measurement can be performed with minimized effect of such nonuniformity. Accordingly, identification accuracy can be improved. Note that the P positions are not necessarily equally spaced.

The direction in which the sensor device 100 or the recording paper M is moved is not limited to the Y-axis direction. For example, the direction in which the sensor device 100 or the recording paper M is moved may be the direction in which the recording paper M is pulled out from the sensor device 100 (i.e., the -X direction in the present example embodiment).

When an operator does not move any one of the sensor device 100 and the recording paper M while the light source 111 is being switched on in the brand identification processes, data is obtained P times at a single detection position.

The printer controller 2090 controls the display unit of the operation panel to display the brand of the recording paper identified by the sensor device 100, and stores the identification results in the RAM of the printer controller 2090.

When the identified brand of the recording paper is displayed on the display unit of the operation panel, an operator returns the sensor device 100 to the originally disposed position. Then, the operator sets the recording paper of the specified brand to the paper feed tray 2060. The brand of the recording paper displayed on the display unit of the operation panel may be registered into the printer controller 2090 by using the keys on the operation panel.

Then, the printer controller 2090 reads the brand of the recording paper from the RAM when a request for a print job is received, and then determines optimal developing conditions and transferring conditions from the development and transfer table for the specified brand of the recording paper.

Then, the printer controller 2090 controls the development device and transfer device of each station in accordance with the determined optimal developing conditions and transferring conditions. For example, the printer controller 2090 controls the transfer voltage or the amount of toner. Accordingly, a high-quality image is formed on recording paper.

As described above, the sensor device 100 according to the present example embodiment includes the optical system 110, the processing device 130, the supporting member 140, the housing 150, and the spring members 170.

The housing 150 has a slit into which recording paper is inserted to identify the brand of the recording paper.

When recording paper is inserted into the slit, the recording paper is sandwiched between the optical system 110 and the supporting member 140. The supporting member 140 is attached to the housing 150 through the spring members 170. When recording paper is fed between the optical system 110 and the supporting member 140, pressing force is applied by the spring members 170 in the +Z-direction of the supporting member 140. By so doing, the recording paper is in full contact with the optical system 110, and the irradiation position or angle of incidence of the light is fixed with reference to the recording paper, and a stable amount of reflection light can be obtained.

The CPU 131 of the processing device 130 measures the output levels of the photosensors when the light source 111 of the optical system 110 is switched on to irradiate to-be-identified recording paper with light, and calculates a relevance ratio R by referring to the brand-specific output level data and using the formula (1) described above. Further, the CPU 131 identifies the brand of the to-be-identified recording paper based on the calculated relevance ratio R.

As the spring members 170, inexpensive metal springs may be used. A variety of such inexpensive metal springs are commercially available and easily accessible. Accordingly, a sensor device can be realized at low cost.

Because a surface emitting laser array is used as a light source in the present example embodiment, a polarizing filter is not required to obtain linearly polarized light as irradiation light. Further, the use of a surface emitting laser array realizes high-density integration of a plurality of light-emitting units, which was difficult to achieve with the conventional LEDs or the like. In such cases, a small-sized light source having a plurality of light-emitting units can be realized. Moreover, the laser light can be all condensed to the optical axis of a collimate lens, and a plurality of rays of light can be collimated with a fixed angle of incidence. In other words, a collimate optical system can be realized at low cost. Accordingly, the cost and size of an optical system can be reduced.

In the brand identification processes, a plurality of light-emitting units of a surface emitting laser array are switched on at the same time. Accordingly, the signal-to-noise ratio (S/N) improves in the output of the photosensors, and identification accuracy also improves. Moreover, the contrast ratio of the speckle pattern of reflection light decreases by switching on all the light-emitting units at the same time, and the amount of reflection light can be measured more accurately. Accordingly, the identification accuracy improves.

Further, the light quantity of the internal reflection light can be increased by switching on the light-emitting units at the same time. Accordingly, it becomes possible to achieve precise separation of the internal reflection light in the optical system 110. Conventionally, such separation of the light reflected from the inside of recording paper was difficult to achieve. The reflection light from the inside of recording paper includes the information about the inside state of the recording paper.

The CPU 131 of the processing device 130 identifies the brand of recording paper based on the signals output from the three photosensors. By taking into consideration the information about the inside state of the recording paper, it becomes possible to improve the level of paper identification such that the level of the recording paper can be determined. Conventionally, such identification of brand was difficult.

Moreover, as it is not necessary to combine various kinds of sensors and the configuration of parts is simple according to the present example embodiment, it is possible to achieve a small sensor device at low cost.

The sensor device 100 according to the present example embodiment can precisely identify an object with simple configuration.

Due to the provision of the sensor device 100, the color printer 2000 according to the present example embodiment can form a high-quality image without increasing the cost or size. Further, troublesome manual settings or failure in printing due to a setting error, which are still present in the conventional products, can be eliminated according to the present example embodiment.

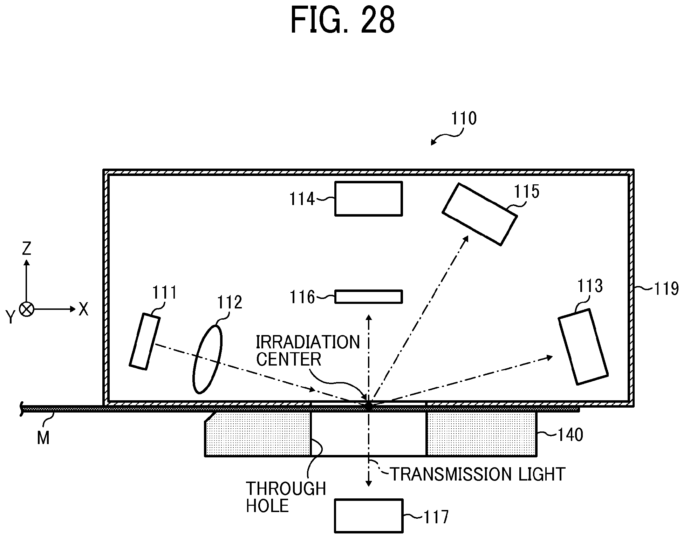

FIG. 27 illustrates a first modification (1) of a sensor device according to an example embodiment of the present invention. FIG. 28 illustrates a first modification (2) of a sensor device according to an example embodiment of the present invention. In the example embodiment described above, as illustrated in FIG. 27 and FIG. 28, a through hole may be made through the supporting member 140 directly below the opening of the dark box 119 (on the -Z-side), and a photosensor 117 may be provided to receive transmission light that has passed through the recording paper M.

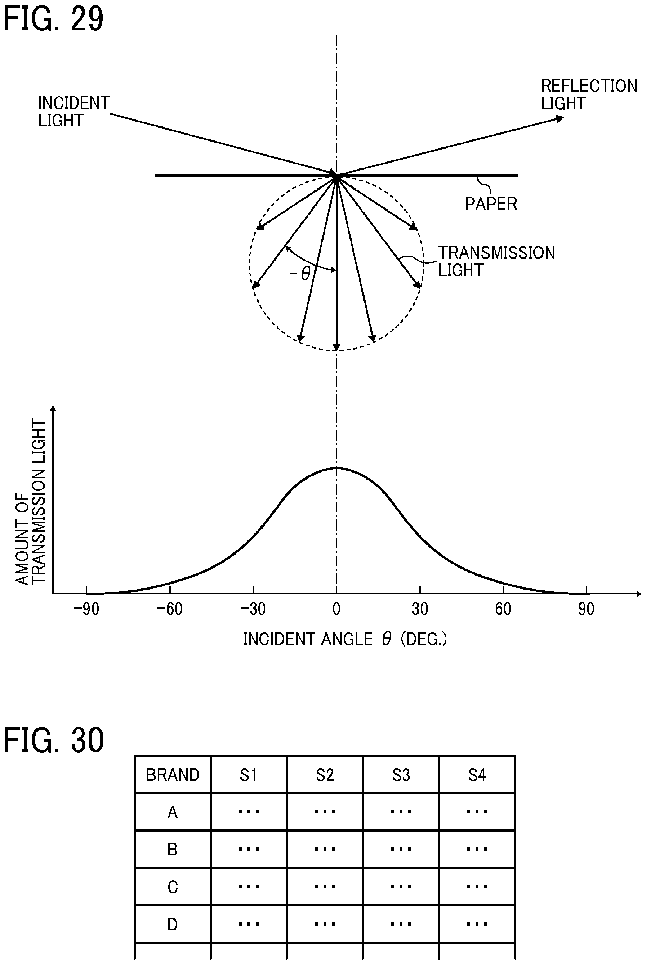

According to the experiments run by the inventor and his associates, it is known that the intensity distribution of the light that passes through the paper follows Lambert's law. FIG. 29 illustrates the characteristics of transmission light according to an example embodiment of the present invention. The light that has entered the paper is dispersed by the fibers of the paper, and has an angle distribution that is not dependent on the angle of incidence, where the peak is at the light intensity of the direction orthogonal to the surface of the paper. For this reason, it is desired that the photosensor 117 be arranged directly underneath the irradiation center.

Because the light that passes through the paper is dispersed by the fibers of the paper, the amount of the transmission light decreases when the paper is thick, i.e., when the basis weight is greater. According to the present example embodiment, the identification accuracy of recording paper can be improved by measuring the amount of the transmission light in consideration of the basis weight of the recording paper, as described above.

In such cases, a relevance ratio R is calculated by using formula (2) below. FIG. 30 illustrates brand-specific output level data that corresponds to the first modification of a sensor device, according to an example embodiment of the present invention. Here, as depicted in FIG. 30, the output level of the photosensor 117 is referred to as S4 in the brand-specific output level data, and a mean value of the output levels of the photosensor 117 when to-be-identified recording paper is irradiated with light is referred to as S4'.

[Formula 2]

$$R = \left(1 - \left|\frac{S1 - S1'}{S1 + S1'}\right|\right) \times \left(1 - \left|\frac{S2 - S2'}{S2 + S2'}\right|\right) \times \left(1 - \left|\frac{S3 - S3'}{S3 + S3'}\right|\right) \times \left(1 - \left|\frac{S4 - S4'}{S4 + S4'}\right|\right) \quad \cdots\cdots (2)$$

In the embodiments described above, cases in which the optical system 110 includes three photosensors for receiving reflection light were described. However, no limitation is indicated therein, and the optical system 110 may include any number of photosensors depending on the desired identification accuracy.

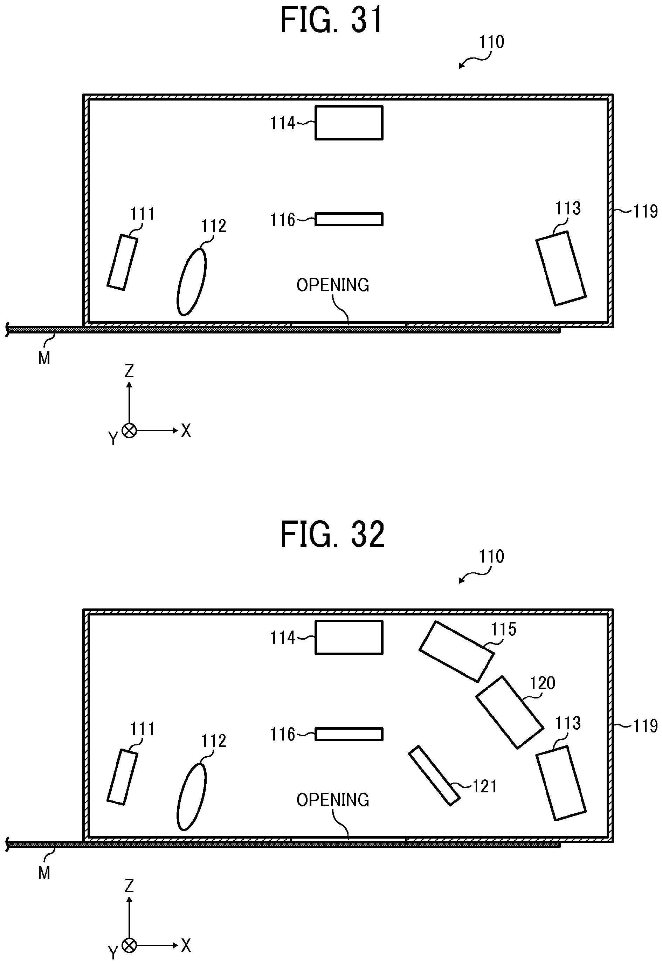

FIG. 31 illustrates a second modification of the sensor device 100 according to an example embodiment of the present invention. In FIG. 31, the optical system 110 according to the example embodiment described above from which the photosensor 115 is removed is illustrated. In such cases, a relevance ratio R is calculated by using formula (3) below.

[Formula 3]

$$R = \left(1 - \left|\frac{S1 - S1'}{S1 + S1'}\right|\right) \times \left(1 - \left|\frac{S2 - S2'}{S2 + S2'}\right|\right) \quad \cdots\cdots (3)$$

FIG. 32 illustrates a third modification (1) of the sensor device 100 according to an example embodiment of the present invention. In FIG. 32, the optical system 110 according to the example embodiment described above to which a photosensor 120 and a polarizing filter 121 are added is illustrated. As illustrated in FIG. 32, the polarizing filter 121 is arranged on the optical path of reflection light including the surface diffuse reflection light and internal reflection light. The polarizing filter 121 transmits the p-polarized light and blocks the s-polarized light. The photosensor 120 is arranged on the optical path of the light that has passed through the polarizing filter 121. The photosensor 120 receives the p-polarized light included in the internal reflection light.

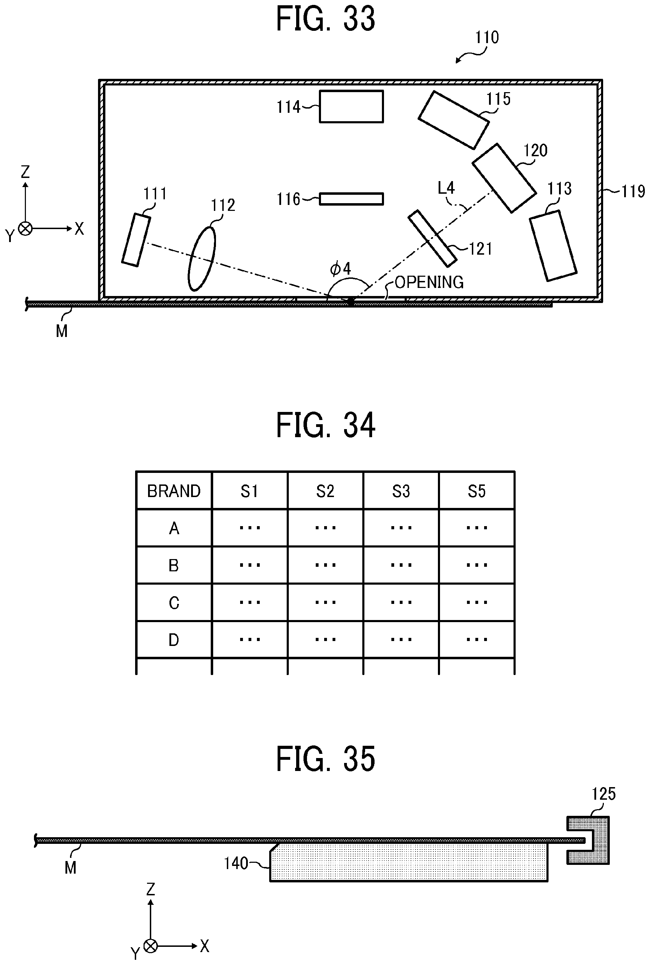

FIG. 33 illustrates a third modification (2) of the sensor device 100 according to an example embodiment of the present invention. As illustrated in FIG. 33, for example, the angle $\varphi 4$ which the surface of the recording paper M forms with a line L4, which is drawn through the irradiation center and the centers of the polarizing filter 121 and photosensor 120, is 150 degrees. Note that the center of the light source 111, the irradiation center, the centers of the polarizing filters, the centers of the photosensors are substantially on the same plane.

In such cases, a relevance ratio R is calculated by using formula (4) below. FIG. 34 illustrates brand-specific output level data that corresponds to the third modification of the sensor device 100, according to an example embodiment of the present invention. Here, as depicted in FIG. 34, the output level of the photosensor 120 is referred to as S5 in the brand-specific output level data, and a mean value of the output levels of the photosensor 120 when to-be-identified recording paper is irradiated with light is referred to as S5'.

[Formula 4]

$$R = \left(1 - \left|\frac{S1 - S1'}{S1 + S1'}\right|\right) \times \left(1 - \left|\frac{S2 - S2'}{S2 + S2'}\right|\right) \times \left(1 - \left|\frac{S3 - S3'}{S3 + S3'}\right|\right) \times \left(1 - \left|\frac{S5 - S5'}{S5 + S5'}\right|\right) \quad \cdots\cdots (4)$$

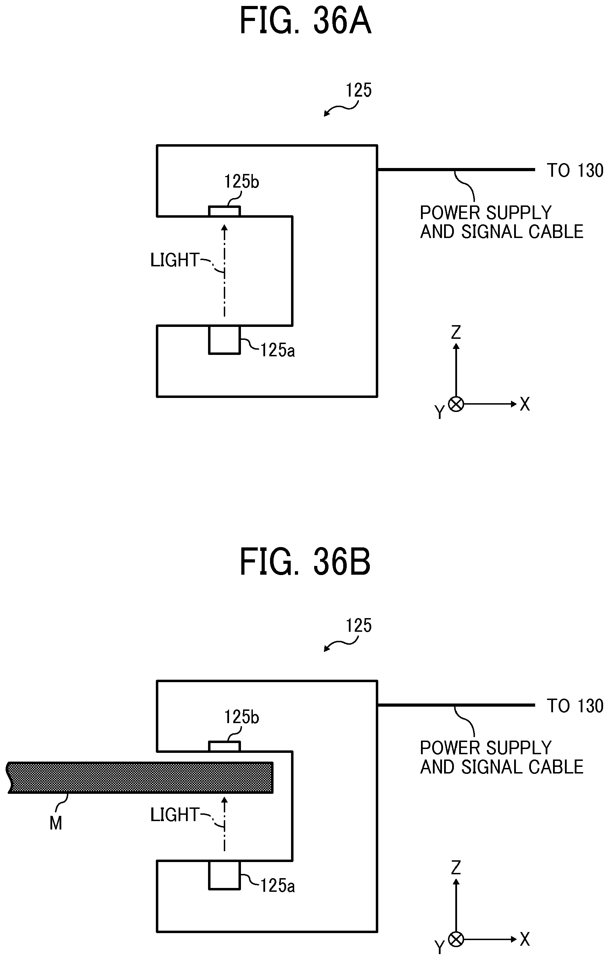

FIG. 35 illustrates a sensor for detecting the insertion of recording paper, according to an example embodiment of the present invention. In the example embodiment described above, as illustrated in FIG. 35, the sensor device 100 may be provided with a sensor 125 that detects the insertion of the recording paper M. As illustrated in FIGS. 36A and 36B, the sensor 125 includes a light emitting element 125a and a light receiving element 125 b that are arranged to face each other in the Z-axis direction. When the light emitted from the light emitting element 125a is received by the light receiving element 125b, it is determined that "no recording paper is inserted". On the other hand, when the light emitted from the light emitting element 125a is not received by the light receiving element 125b, it is determined that "recording paper is inserted". In other words, the sensor 125 is a so-called transmission photo interrupter sensor. However, a different type of sensor may also be used. In other words, any sensor may be used as long as that sensor can detect the insertion of recording paper.

In the example embodiment described above, the light source 111 and the related processes may be controlled as follows. The light source 111 is switched on and the output of the photosensors is obtained when it is determined that "recording paper is inserted". On the other hand, the light source 111 is switched off and data processing is performed when it is determined that "no recording paper is inserted". As described above, it can be configured such that measurement will be performed only when recording paper is inserted into the slit. Accordingly, measurement is not erroneously performed when no recording paper is inserted. Moreover, it may be configured such that measurement will start automatically without any instruction through the operation panel, or power consumption may be reduced by minimizing the length of time during which the light source 111 is switched on.

Moreover, in the example embodiment described above, the sensor device 100 may be provided with an LED that illuminates when it is determined that "recording paper is inserted". Due to such provision of an LED, an operator can visually notice that recording paper has been inserted.

In the example embodiment described above, at least some of the processes performed by a program, which is executed by the CPU 131, may be performed by hardware, or all of the processes may be performed by hardware.

Note that cases in which recording paper is irradiated with the s-polarized light have been described in the example embodiment described above, but no limitation is indicated therein. In other words, recording paper may be irradiated with the p-polarized light. In such a case, however, a polarizing filter that transmits the s-polarized light is used in place of the polarizing filter 116, and the photosensor 114 receives the s-polarized light included in the internal reflection light.

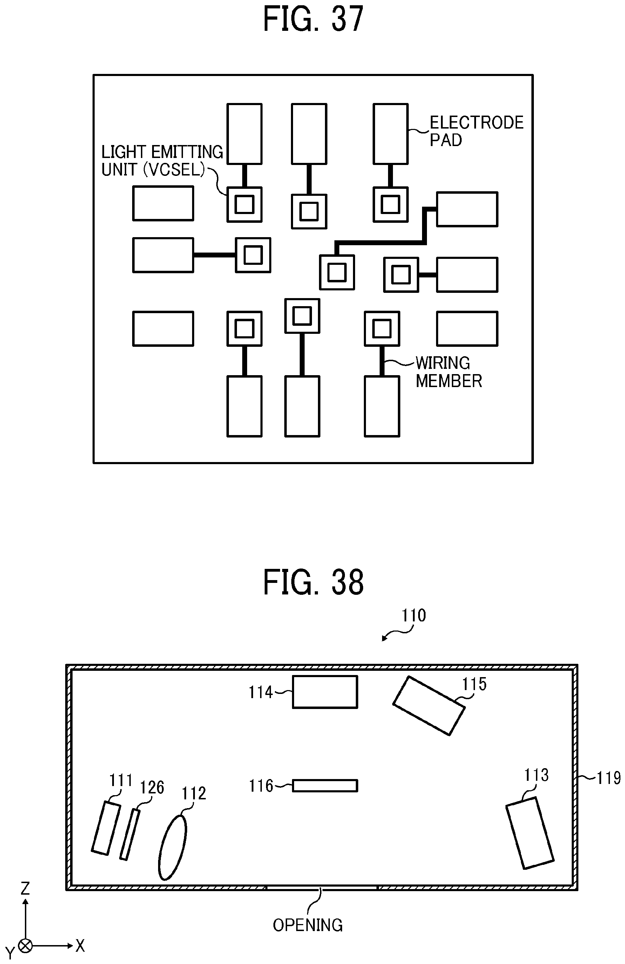

FIG. 37 illustrates a modification of a surface emitting laser array according to an example embodiment of the present invention. As illustrated in FIG. 37, at least some of light-emitting units of a surface emitting laser array may be spaced differently from the other light-emitting units. In other words, the space between adjacent light-emitting units may vary.

In the example embodiment described above, cases in which the light source 111 includes nine light-emitting units were described. However, no limitation is indicated therein.

In the example embodiment described above, a conventional laser diode (LD) may be used in place of the surface emitting laser array.

In the example embodiment described above, cases in which the light source 111 emits linearly polarized light were described. However, no limitation is indicated therein.

FIG. 38 illustrates a fourth modification of the sensor device 100 according to an example embodiment of the present invention. In the example embodiment described above, as illustrated in FIG. 38, a polarizing filter 126 is provided to obtain s-polarized light from the light emitted from the light source 111.

In the example embodiment described above, a different kind of elastic member may be used in place of the spring members 170.

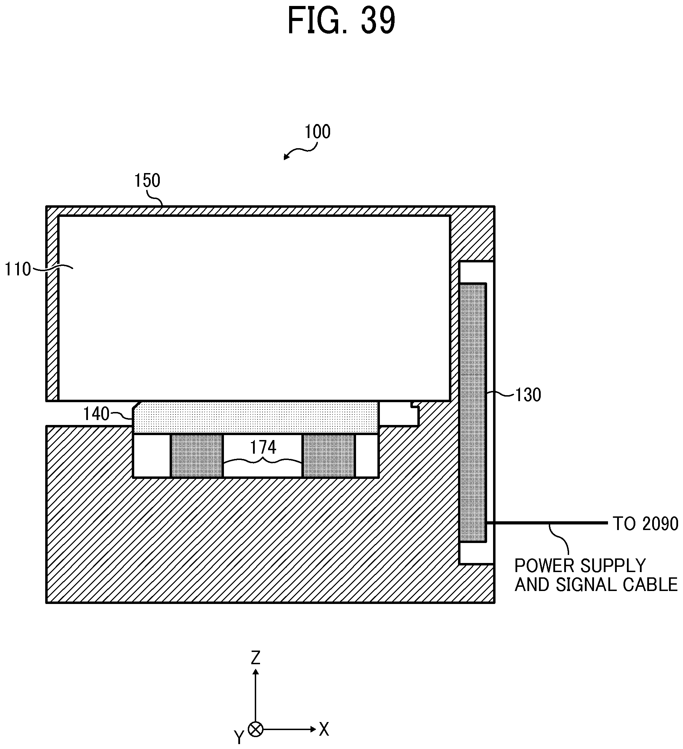

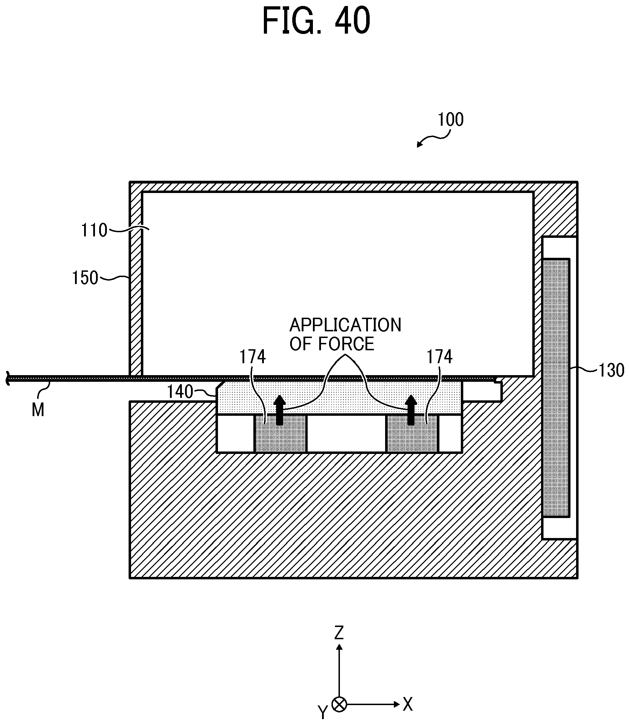

FIG. 39 illustrates a fifth modification (1) of the sensor device 100 according to an example embodiment of the present invention. FIG. 40 illustrates a fifth modification (2) of the sensor device 100 according to an example embodiment of the present invention. As illustrated in FIGS. 39 and 40, for example, a plurality of rubber members 174 may be used in place of the spring members 170. Moreover, a porous resin member such as a sponge may be used in place of the spring members 170.

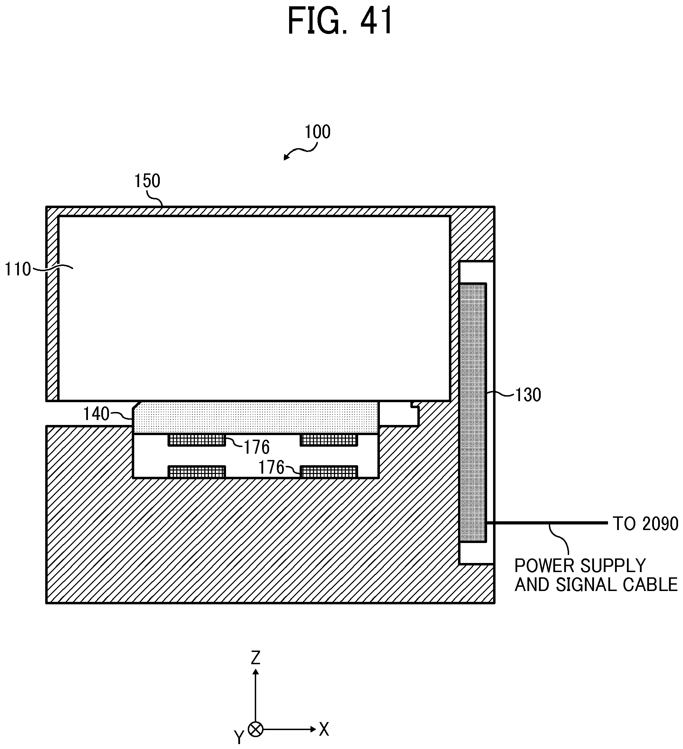

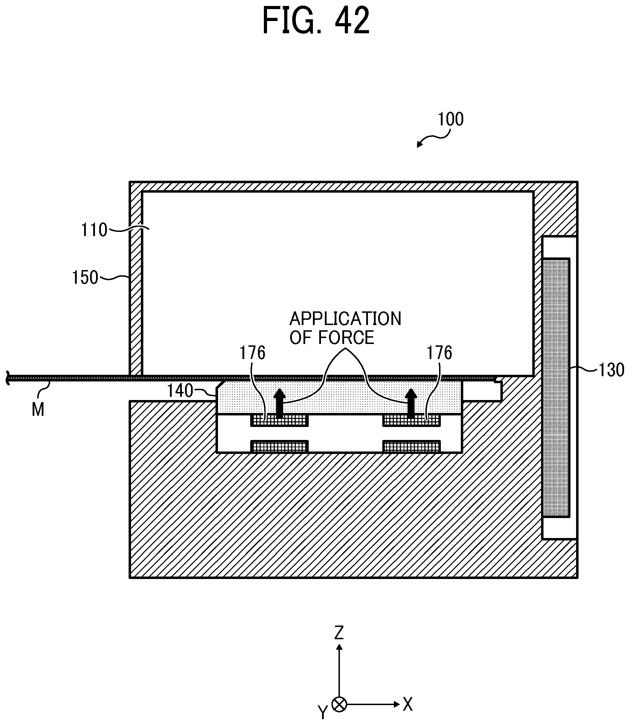

FIG. 41 illustrates a sixth modification (1) of the sensor device 100 according to an example embodiment of the present invention. FIG. 42 illustrates a sixth modification (2) of the sensor device 100 according to an example embodiment of the present invention. In the example embodiment described above, as illustrated in FIGS. 41 and 42, for example, a plurality of magnets 176 may be used in place of the spring members 170. In the present modification, the repulsive force caused between two magnets that are disposed in the Z-axis direction is used. There is some concern that spring members or rubber members become deteriorated due to aging and become incapable of appropriately maintaining the holding force. For this reason, long-term use of spring members and rubber members is to be avoided. By contrast, repulsive force does not deteriorate easily when magnets are used. For this reason, magnets are suitable for long-term use. Magnets may be arranged to use the attractive force between two magnets.

Note that cases in which the supporting member 140 is pressed down by recording paper when the recording paper is inserted into the slit of the housing 150 were described in the example embodiment described above, but no limitation is indicated therein. It may be configured such that the dark box 119 is lifted up by recording paper.

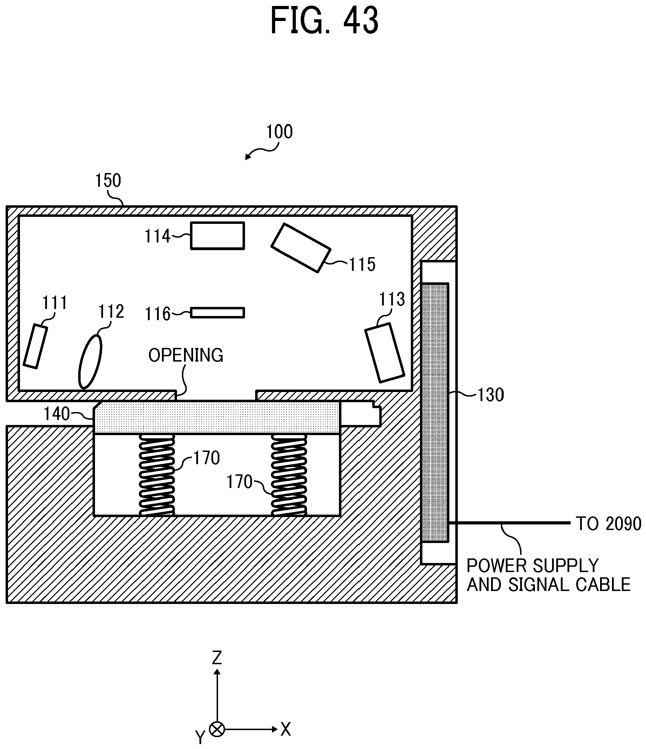

FIG. 43 illustrates a seventh modification (1) of the sensor device 100 according to an example embodiment of the present invention. In the example embodiment described above, the dark box 119 may be omitted as illustrated in FIG. 43.

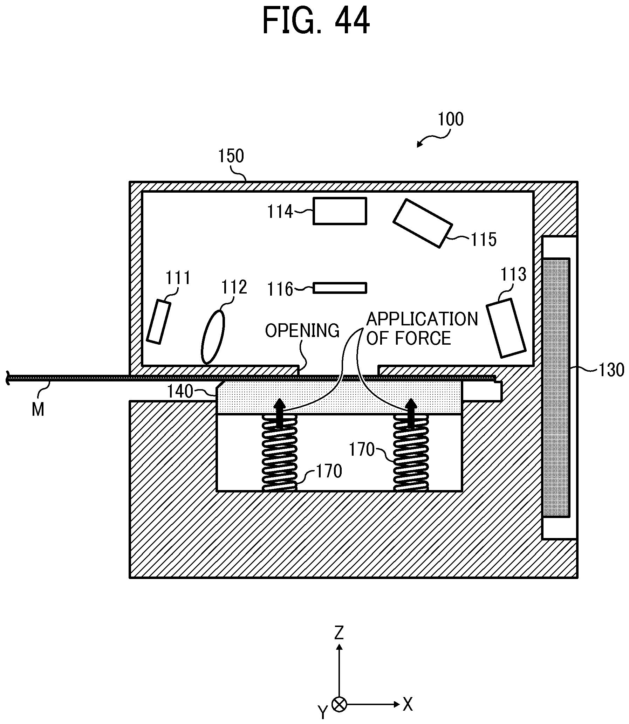

FIG. 44 illustrates a seventh modification (2) of the sensor device 100 according to an example embodiment of the present invention. In the modification described above with reference FIG. 43, the housing 150 does the job of the -Z-side surface of the dark box 119, as illustrated in FIG. 44.

In the example embodiment described above, it is desired that a condensing lens be provided in front of each of the photosensors. By so doing, the changes in the amount of the light received at photosensors can be reduced.

In the example embodiment described above, cases in which the processing device 130 performs brand identification processes were described, but no limitation is indicated therein. For example, the printer controller 2090 may perform brand identification processes. In that case, the brand-specific output level data may be stored in a ROM of the printer controller 2090.

In the example embodiment described above, cases in which the light source 111 illuminates for three seconds in the brand identification processes were described. However, no limitation is indicated therein. The length of time during which the light source 111 illuminates in the brand identification processes may be set through the operation panel.

In the example embodiment described above, a start button for the identification process may be provided for the sensor device 100. In that case, it is not necessary for an operator to input a request for an identification process through the operation panel.

In the example embodiment described above, an LED that indicates the on/off of the light source 111 may be provided for the sensor device 100. Due to such provision of an LED, an operator can visually know the on/off of the light source 111.

In the example embodiment described above, a display unit may be provided for the sensor device 100. In that case, the processing device 130 controls the display unit to display the identification results.

In the example embodiment described above, cases in which the outside shape of the housing 150 of the sensor device 100 is like a block were described. However, no limitation is indicated therein. For example, the outside shape of the housing 150 may have a curve like a mouse. In other words, the housing 150 may have any outside shape as long as the housing 150 has a slit of prescribed length in the direction in which the recording paper is inserted.

In the example embodiment described above, an object to be identified by the sensor device 100 is not limited to recording paper.

Note that cases in which recording paper is pressed and held such that the relative positions of the recording paper inserted into a slit and a plurality of photosensors are fixed were described in the example embodiment described above, but no limitation is indicated therein. For example, the recording paper may be held by electrostatic force instead of the pressing force, or the recording paper may be held by suction (negative pressure).

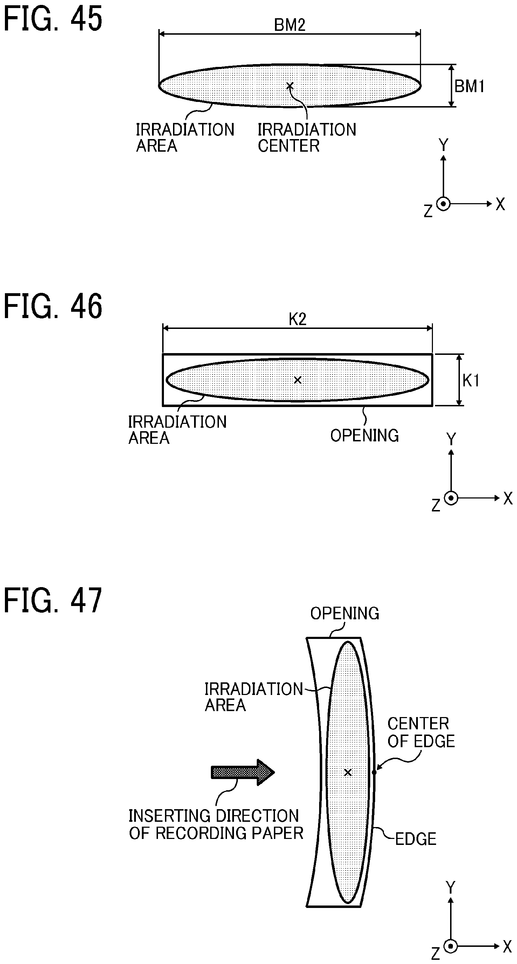

FIG. 45 illustrates an irradiation area according to an example embodiment of the present invention. In the example embodiment described above, as illustrated in FIG. 45, the shape of an irradiation area is elliptic where the major (longer) axis is the X axis and the minor (shorter) axis is the Y axis. The length of the minor axis (BM1) is 2.3 mm, and the length of the major axis (BM2) is 13.4 mm. Preferably, the opening made on the dark box 119 is made along the circumference of the irradiation area and the size is as small as possible, as long as the opening does not cover the irradiation area.

FIG. 46 illustrates a desired shape of an opening according to an example embodiment of the present invention. In the example embodiment described above, some margin is provided in consideration of tolerance or adjustment of beam, and a preferred shape of the opening is, as illustrated in FIG. 46, a rectangle where the length in the Y-axis direction (K1) is 4 mm and the length in the X-axis direction (K2) is 15 mm. In other words, the opening is shaped to have the longitudinal direction parallel to the direction in which a light source and a plurality of photosensors are arranged (i.e., X-axis direction).

If the opening is narrower than the irradiation area and some of the irradiation area is covered by the -Z-side surface of the dark box 119, the light reflected at the -Z-side surface of the dark box 119 become stray light, and such stray light may be received by a photosensor. In that case, the signal-to-noise ratio (S/N) of the signal output from the photosensor decreases, and the identification accuracy deteriorates.

By contrast, if the opening is much wider than the irradiation area, dust or paper dust may enter the dark box 119 and stick to a photosensor. If dust or paper dust sticks to a photosensor, the amount of the light received at the photosensor varies, and the identification accuracy deteriorates. Moreover, if the opening is too wide, bending of recording paper may occur, and such bending makes it difficult to measure the reflected light with precision.

If the reduction in identification accuracy is no concern, the opening may be shaped differently from the preferred configuration described above. As long as an opening is arranged on a part of the -Z-side surface of the dark box 119, recording paper can be held by the -Z-side surface of the dark box 119.

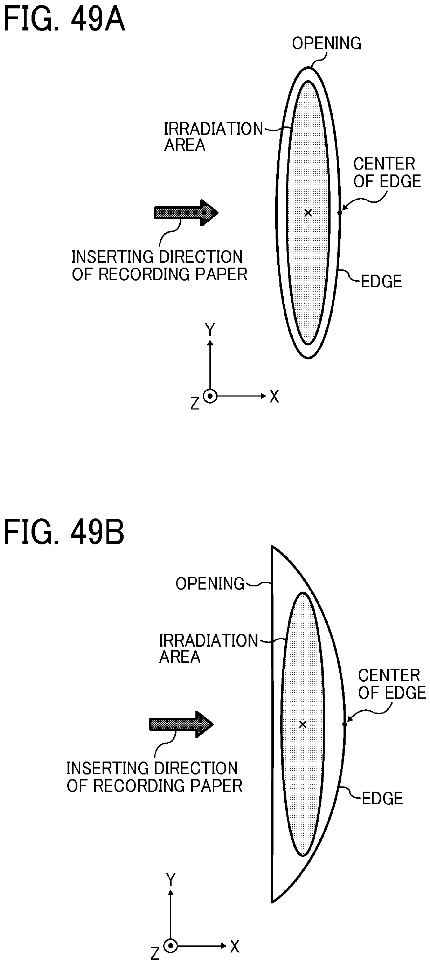

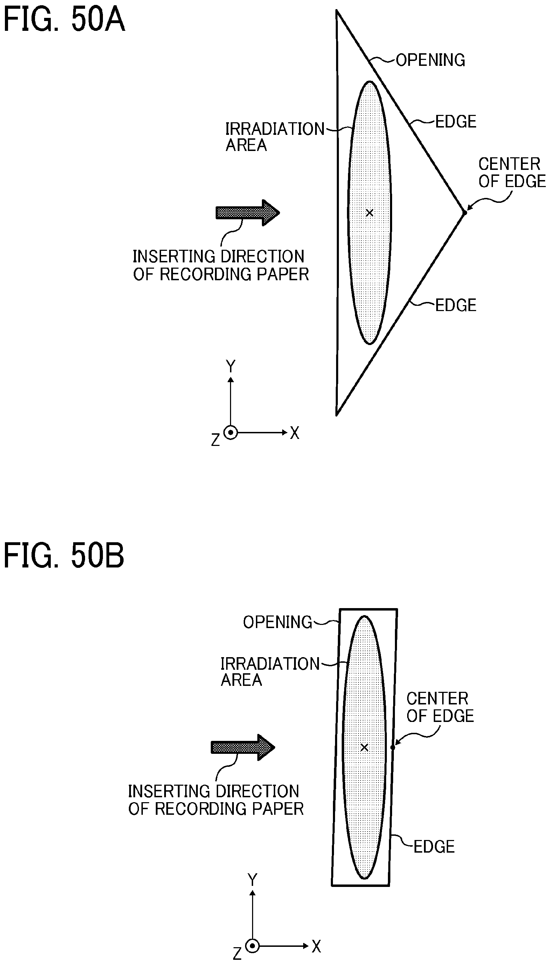

FIG. 47 illustrates a first modification of an opening according to an example embodiment of the present invention. When there is some concern that recording paper is caught at an edge of the opening, as illustrated in FIG. 47 (and FIGS. 49A and 49B and FIGS. 50A and 50B as will be described later), in the first modification of the opening, the center of the edge that is on the leading-end side of the direction in which the recording paper is inserted (i.e., on the +X side) is positioned on a further leading-end side of the insertion direction than at least one of the two ends of the above edge. Accordingly, it becomes possible to prevent the recording paper from being caught at an edge of the opening.

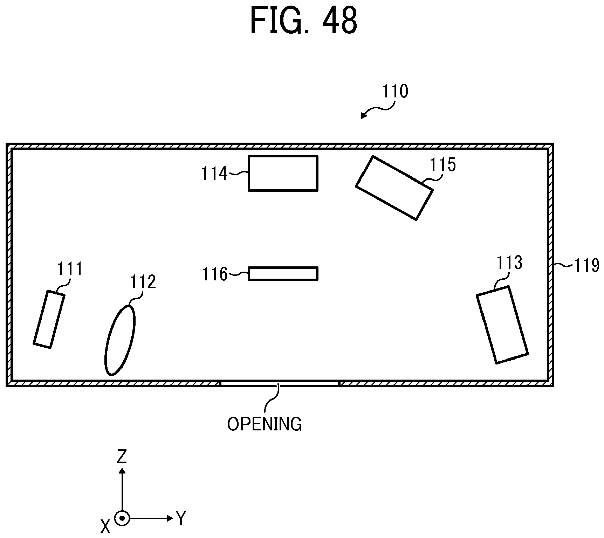

FIG. 48 illustrates the arrangement of a light source and a plurality of photosensors with reference to an opening of a modification, according to an example embodiment of the present invention. Note that, as illustrated in FIG. 48, the direction in which light source and a plurality of photosensors are arranged is the Y-axes direction, which is different from the example embodiment described above.

In FIG. 47, the above-described edge of the opening is convexly curved towards the direction in which the recording paper is inserted, and the center of the above-described edge is positioned on a further leading-end side of the insertion direction than at least one of the two ends of the edge.

FIG. 49A illustrates a second modification of an opening according to an example embodiment of the present invention. As illustrated in FIG. 49A, in the second modification of the opening, the shape of the opening is elliptic. Moreover, the above-described edge of the opening is convexly curved towards the direction in which the recording paper is inserted, and the center of the above-described edge is positioned on a further leading-end side of the insertion direction than at least one of the two ends of the edge.

FIG. 49B illustrates a third modification of an opening according to an example embodiment of the present invention. As illustrated in FIG. 49B, in the third modification of the opening, the shape of the opening is semiellptic. Moreover, the above-described edge of the opening is convexly curved towards the direction in which the recording paper is inserted, and the center of the above-described edge is positioned on a further leading-end side of the insertion direction than at least one of the two ends of the edge.

FIG. 50A illustrates a fourth modification of an opening according to an example embodiment of the present invention. As illustrated in FIG. 50A, in the fourth modification of the opening, the shape of the opening is triangular. Moreover, the above-described edge is composed of two sides of a triangle, and the point of intersection of these two sides is the center of the edge. The center of the above-described edge is positioned on a further leading-end side of the insertion direction than at least one of the two ends of the edge.

FIG. 50B illustrates a fifth modification of an opening according to an example embodiment of the present invention. As illustrated in FIG. 50B, in the fifth modification of the opening, the opening is shaped like a parallelogram. Moreover, the above-described edge of the opening is a straight line that is inclined with reference to the direction orthogonal to the direction in which the recording paper is inserted, and the center of the above-described edge is positioned on a further leading-end side of the insertion direction than at least one of the two ends of the edge (i.e., the end on the -Y side).

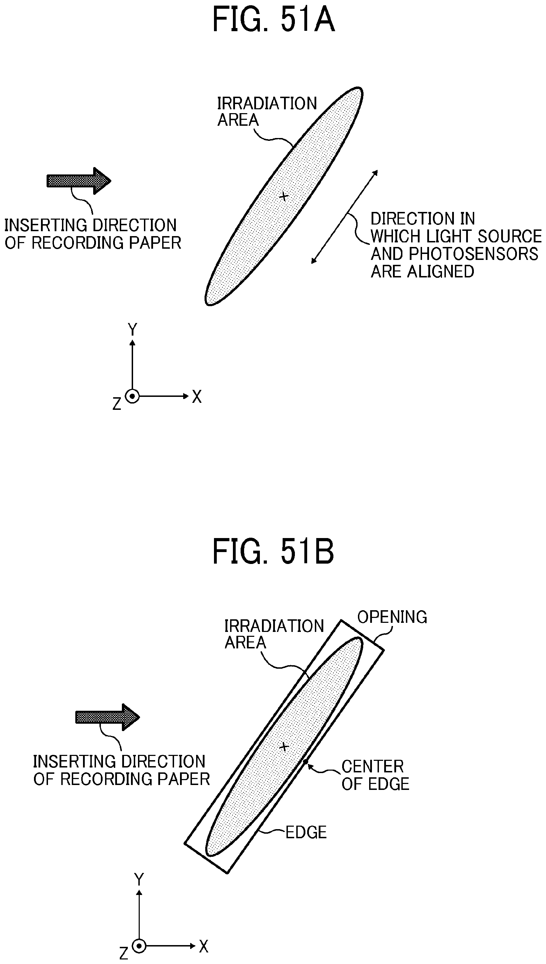

FIGS. 51A and 51B illustrate other methods of preventing recording paper from being caught at an edge of an opening, according to an example embodiment of the present invention. As illustrated in FIG. 51A for example, the direction in which light source and a plurality of photosensors are arranged may be inclined with reference to the direction in which the recording paper is inserted. In that case, as illustrated in FIG. 51B, even if the opening is rectangular, it becomes possible to prevent the recording paper from being caught at an edge of the opening. Moreover, the above-described edge of the opening is the long side of the parallelogram on the +X side, and the center of the above-described edge is positioned on a further leading-end side of the insertion direction than at least one of the two ends of the edge (i.e., the end on the -Y side).

In the example embodiment described above, cases in which the color printer 2000 is used as an image forming apparatus were described, but no limitation is indicated therein. For example, a laser printer that forms monochrome images may be used as an image forming apparatus. Moreover, an image forming apparatus other than a printer, for example, a copier, a facsimile, or a multifunction peripheral (MFP) into which these elements are integrated, may be used.

In the example embodiment described above, cases in which an image forming apparatus includes four photoreceptor drums were described, but no limitation is indicated therein. For example, a printer for which five photoreceptor drums are provided may be used as an image forming apparatus.

In the example embodiment described above, cases of an image forming apparatus in which a toner image is transferred from a photoreceptor drum to a transfer belt and then is transferred to recording paper were described, but no limitation is indicated therein. For example, an image forming apparatus in which a toner image is directly transferred from a photoreceptor drum to recording paper may be used.

The sensor device 100 may be applied to an image forming apparatus in which an image is formed by ejecting ink onto recording paper.

Numerous additional modifications and variations are possible in light of the above teachings. It is therefore to be understood that within the scope of the appended claims, the disclosure of the present invention may be practiced otherwise than as specifically described herein.

**Claims**

1. An image forming apparatus (2000) comprising an image forming unit (2000) configured to form an image on a recording medium (M); an adjustment unit (130, 2090) configured to adjust a condition for image formation, based on an output from a sensor device (100); a printer housing 2200; an operation panel; and the sensor device comprising:

    a light source (111);

a photo-detection system (110) configured to receive light emitted from the light source (111) and reflected at a sheet-like object (M), whereby such a recording medium (M) is the object (M); and

a holding member (140, 170, 174, 176) configured to hold the object (M) such that relative positions of a surface of the object (M) inserted into the sensor device (100) and the photo-detection system (110) are fixed;

the sensor device (100) further comprises a housing (150), having an inner space accommodating the light source (111), the photo-detection system (110) and the holding member (140, 170, 174, 176);

**characterized in that:**

the sensor device (100) is detached from the printer housing and is disposed near an operation panel, such that an operator can pick it up with his/her hand;

the housing (150) of the sensor device (100) comprises a -X side surface, a -Y side surface and a +Y side surface in a XYZ Cartesian coordinate system, and has a slit into which the object (M) is inserted, the slit being formed in the -X side surface, having a prescribed length in the X-direction and penetrating to the -Y side surface and the +Y side surface of the housing; and

the sensor device (100) is configured such that the sheet-like object (M) can be inserted into the slit in a + X-direction by an operator holding the sheet-like object (M) with both hands and applying tension in the +Y and -Y directions to the sheet-like object (M).

2. The image forming apparatus (2000) according to claim 1, wherein
the holding member (140, 170, 174, 176) is a pressing member (170, 174, 176) configured to indirectly press the object (M) inserted in the slit in a direction orthogonal to a surface of the object (M).

3. The image forming apparatus (2000) according to claim 2, further comprising:

a supporting member (140) disposed inside the housing (150) and configured to support the object (M) inserted into the slit,

wherein the pressing member (170, 174, 176) presses the supporting member (140) such that a surface of the object (M) is positioned at a specified position with reference to the light source (111) and the photo-detection system (110).

4. The image forming apparatus (2000) according to claim 3, wherein
the specified position is within the slit with reference to a direction orthogonal to the surface of the object (M).

5. The image forming apparatus (2000) according to claim 3 or 4, wherein
the supporting member (140) has a guide unit configured to guide the object (M) when the object (M) is inserted into the slit, and
the guide unit has at least one of an inclined plane surface and an inclined curved surface.

6. The image forming apparatus (2000) according to any one of claims 3 to 5, wherein:

the supporting member (140) has at least one of a through hole and a light transmission member transmitting light passed through the object (M); and

the photo-detection system (110) receives light transmitted through at least one of the through hole and the light transmission member.

7. The image forming apparatus (2000) according to any one of claims 2 to 6, wherein the pressing member (170, 174, 176) presses the object (M) inserted into the slit so as not to restrict relative movement of the object (M) in the housing (150) by controlling pressing force.

8. The image forming apparatus (2000) according to any one of claims 1 to 7, further comprising: a sensor (125) configured to detect insertion of the object (M) into the slit.

9. The image forming apparatus (2000) according to any one of claims 1 to 8, wherein:

the housing (150) of the sensor device (100) has a wall (119) disposed on a side of the slit having the light source (111) and the photo-detection system (110), the wall (119) touching the object (M) when the object (M) is inserted into the slit; and

the wall (119) has an opening passing light emitted from the light source (111).

10. The image forming apparatus (2000) according to claim 9, wherein the opening is shaped to have a longitudinal direction parallel to a direction in which the light source (111) and the photo-detection system (110) are disposed.

11. The image forming apparatus (2000) according to claim 9 or 10, wherein the opening has an edge on a leading-end side of an insertion direction of the object (M), and the edge has a center on a further leading-end side of the insertion direction than at least one of two ends of the edge.

12. The image forming apparatus (2000) according to any one of claims 1 to 11, wherein:

the light source (111) emits linearly polarized light having a first polarization direction; and
the photo-detection system (110) comprises

an optical element (116) disposed on an optical path of light reflected by diffuse reflection from an incidence plane of the object (M) and configured to transmit linearly polarized light of a second polarization direction orthogonal to the first polarization direction,
a first photodetector (113) disposed on an optical path of light reflected at the object (M) by regular reflection, and
a second photodetector (114) configured to detect light transmitted through the optical element (116).

13. The image forming apparatus (2000) according to claim 12, wherein the photo-detection system (110) comprises: a third photodetector disposed on an optical path of light reflected by diffuse reflection from an incidence plane of the object (M).

14. The image forming apparatus (2000) according to any preceding claim, wherein the adjustment unit (130, 2090) identifies the recording medium based on an output from the sensor device (100), and adjusts a condition for image formation based on a result of identification.


**Patentansprüche**

1. Bilderzeugungsvorrichtung (2000), umfassend eine Bilderzeugungseinheit (2000), die konfiguriert ist, um ein Bild auf einem Aufzeichnungsmedium (M) auszubilden; eine Einstelleinheit (130, 2090), die konfiguriert ist, um einen Zustand für eine Bilderzeugung auf Grundlage einer Ausgabe von einer Sensorvorrichtung (100) einzustellen; ein Druckergehäuse (2200); ein Bedienfeld; und wobei die Sensorvorrichtung umfasst:

eine Lichtquelle (111);
ein Fotoerfassungssystem (110), das konfiguriert ist, um von der Lichtquelle (111) emittiertes und an einem bogenartigen Objekt (M) reflektiertes Licht zu empfangen, wodurch ein solches Aufzeichnungsmedium (M) das Objekt (M) ist; und
ein Halteelement (140, 170, 174, 176), das konfiguriert ist, um das Objekt (M) derart zu halten, dass die relativen Positionen einer Oberfläche des Objekts (M), das in die Sensorvorrichtung (100) und das Fotoerfassungssystem (110) eingeführt wird, fixiert sind;
wobei die Sensorvorrichtung (100) ferner ein Gehäuse (150) umfasst, das einen Innenraum aufweist, der die Lichtquelle (111), das Fotoerfassungssystem (110) und das Halteelement (140, 170, 174, 176) aufnimmt;
**dadurch gekennzeichnet, dass:**

die Sensorvorrichtung (100) von dem Druckergehäuse gelöst ist und in der Nähe eines Bedienfeldes derart angeordnet ist, dass ein Bediener sie mit seiner Hand aufheben kann;
das Gehäuse (150) der Sensorvorrichtung (100) eine -X-Seitenoberfläche, eine -Y-Seitenoberfläche und eine +Y-Seitenoberfläche in einem kartesischen XYZ-Koordinatensystem umfasst und einen Schlitz aufweist, in den das Objekt (M) eingeführt wird, wobei der Schlitz in der -X-Seitenoberfläche ausgebildet ist, eine vorgeschriebene Länge in der X-Richtung aufweist und zu der -Y-Seitenoberfläche und der +Y-Seitenoberfläche des Gehäuses durchdringt; und
die Sensorvorrichtung (100) derart konfiguriert ist, dass das bogenartige Objekt (M) in den Schlitz in einer +X-Richtung durch einen Bediener eingeführt werden kann, der das bogenartige Objekt (M) mit beiden Händen hält und eine Spannung in der +Y- und -Y-Richtung auf das blattartige Objekt (M) ausübt.

2. Bilderzeugungsvorrichtung (2000) nach Anspruch 1, wobei

das Halteelement (140, 170, 174, 176) ein Druckelement (170, 174, 176) ist, das konfiguriert ist, um das in den Schlitz eingeführte Objekt (M) indirekt in einer Richtung orthogonal zu einer Oberfläche des Objekts (M) zu drücken.

3. Bilderzeugungsvorrichtung (2000) nach Anspruch 2, ferner umfassend:

ein Stützelement (140), das in dem Inneren des Gehäuses (150) angeordnet ist und konfiguriert ist, um das in den Schlitz eingeführte Objekt (M) zu stützen,
wobei das Druckelement (170, 174, 176) das Stützelement (140) derart drückt, dass eine Oberfläche des Objekts (M) in einer bestimmten Position in Bezug auf die Lichtquelle (111) und das Fotoerfassungssystem (110) positioniert wird.

4. Bilderzeugungsvorrichtung (2000) nach Anspruch 3, wobei
die bestimmte Position innerhalb des Schlitzes in Bezug auf eine Richtung orthogonal zu der Oberfläche des Objekts (M) liegt.

5. Bilderzeugungsvorrichtung (2000) nach Anspruch 3 oder 4, wobei das Stützelement (140) eine Führungseinheit aufweist, die konfiguriert ist, um das Objekt (M) zu führen, wenn das Objekt (M) in den Schlitz eingeführt wird, und die Führungseinheit wenigstens eine einer geneigten ebenen Oberfläche und einer geneigten gekrümmten Oberfläche aufweist.

6. Bilderzeugungsvorrichtung (2000) nach einem der Ansprüche 3 bis 5, wobei:

das Stützelement (140) wenigstens eines eines Durchgangslochs und eines Lichtübertragungselements aufweist, das durch das Objekt (M) passierte Licht überträgt; und
das Fotoerfassungssystem (110) Licht, das durch wenigstens eines des Durchgangslochs und des Lichtübertragungselements übertragen wird, empfängt.

7. Bilderzeugungsvorrichtung (2000) nach einem der Ansprüche 2 bis 6, wobei das Druckelement (170, 174, 176) das in den Schlitz eingeführte Objekt (M) derart drückt, dass die relative Bewegung des Objekts (M) in dem Gehäuse (150) durch das Steuern der Druckkraft nicht eingeschränkt wird.

8. Bilderzeugungsvorrichtung (2000) nach einem der Ansprüche 1 bis 7, ferner umfassend: einen Sensor (125), der konfiguriert ist, um das Einführen des Objekts (M) in den Schlitz zu erfassen.

9. Bilderzeugungsvorrichtung (2000) nach einem der Ansprüche 1 bis 8, wobei:

das Gehäuse (150) der Sensorvorrichtung (100) eine Wand (119) aufweist, die auf einer Seite des Schlitzes mit der Lichtquelle (111) und dem Fotoerfassungssystem (110) angeordnet ist, wobei die Wand (119) das Objekt (M) berührt, wenn das Objekt (M) in den Schlitz eingeführt wird; und
die Wand (119) eine Öffnung aufweist, die von der Lichtquelle (111) emittiertes Licht passiert.

10. Bilderzeugungsvorrichtung (2000) nach Anspruch 9, wobei die Öffnung geformt ist, um eine Längsrichtung parallel zu einer Richtung aufzuweisen, in der die Lichtquelle (111) und das Fotoerfassungssystem (110) angeordnet sind.

11. Bilderzeugungsvorrichtung (2000) nach Anspruch 9 oder 10, wobei die Öffnung eine Kante an einer vorderen Endseite einer Einführungsrichtung des Objekts (M) aufweist und die Kante eine Mitte an einer weiter entfernten vorderen Endseite der Einführungsrichtung als wenigstens eines von zwei Enden der Kante aufweist.

12. Bilderzeugungsvorrichtung (2000) nach einem der Ansprüche 1 bis 11, wobei:

die Lichtquelle (111) linear polarisiertes Licht mit einer ersten Polarisationsrichtung emittiert; und
das Fotoerfassungssystem (110) umfasst:

ein optisches Element (116), das auf einem optischen Pfad von Licht angeordnet ist, das durch eine diffuse Reflexion von einer Einfallsebene des Objekts (M) reflektiert wird, und konfiguriert ist, um linear polarisiertes Licht einer zweiten, zu der ersten Polarisationsrichtung orthogonalen Polarisationsrichtung zu übertragen, einen ersten Fotodetektor (113), der auf einem optischen Pfad von Licht angeordnet ist, das an dem Objekt (M) durch eine regelmäßige Reflexion reflektiert wird, und

einen zweiten Fotodetektor (114), der konfiguriert ist, um das durch das optische Element (116) übertragene Licht zu erfassen.

13. Bilderzeugungsvorrichtung (2000) nach Anspruch 12, wobei das Fotoerfassungssystem (110) umfasst: einen dritten Fotodetektor, der auf einem optischen Pfad von Licht angeordnet ist, das durch die diffuse Reflexion von einer Einfallsebene des Objekts (M) reflektiert wird.

14. Bilderzeugungsvorrichtung (2000) nach einem vorhergehenden Anspruch, wobei die Einstelleinheit (130, 2090) das Aufzeichnungsmedium auf Grundlage einer Ausgabe von der Sensorvorrichtung (100) identifiziert und einen Zustand für die Bilderzeugung auf Grundlage eines Ergebnisses der Identifizierung einstellt.

**Revendications**

1. Appareil de formation d'image (2000), comprenant une unité de formation d'image (2000) configurée pour former une image sur un support d'enregistrement (M) ; une unité d'ajustement (130, 2090) configurée pour ajuster une condition pour formation d'image, sur la base d'une sortie provenant d'un dispositif capteur (100) ; un logement d'imprimante (2200) ; un panneau de commande ; et le dispositif capteur comprenant :

   une source de lumière (111) ;
   un système de photo-détection (110) configuré pour recevoir de la lumière émise de la source de lumière (111) et réfléchie sur un objet en forme de feuille (M), moyennant quoi un tel support d'enregistrement (M) est l'objet (M) ; et
   un organe de retenue (140, 170, 174, 176) configuré pour retenir l'objet (M) de telle sorte que des positions relatives d'une surface de l'objet (M) inséré dans le dispositif capteur (100) et du système de photo-détection (110) soient fixes ;
   le dispositif capteur (100) comprend en outre un logement (150), ayant un espace intérieur logeant la source de lumière (111), le système de photo-détection (110) et l'organe de retenue (140, 170, 174, 176) ;
   **caractérisé en ce que :**

   le dispositif capteur (100) est détaché du logement d'imprimante et est disposé près d'un panneau de commande, de telle sorte qu'un opérateur puisse le saisir avec sa main ;
   le logement (150) du dispositif capteur (100) comprend une surface de côté -X, une surface de côté -Y et une surface de côté +Y dans un système de coordonnées cartésiennes XYZ, et a une fente dans laquelle l'objet (M) est inséré, la fente étant formée dans la surface de côté -X, ayant une longueur prescrite dans la direction X et pénétrant jusqu'à la surface de côté -Y et la surface de côté +Y du logement ; et
   le dispositif capteur (100) est configuré de telle sorte que l'objet en forme de feuille (M) puisse être inséré dans la fente dans une direction +X par un opérateur retenant l'objet en forme de feuille (M) avec les deux mains et appliquant une tension dans les directions +Y et -Y sur l'objet en forme de feuille (M).

2. Appareil de formation d'image (2000) selon la revendication 1, dans lequel l'organe de retenue (140, 170, 174, 176) est un organe de pression (170, 174, 176) configuré pour indirectement presser l'objet (M) inséré dans la fente dans une direction orthogonale à une surface de l'objet (M).

3. Appareil de formation d'image (2000) selon la revendication 2, comprenant en outre :

   un organe de support (140) disposé à l'intérieur du logement (150) et configuré pour supporter l'objet (M) inséré dans la fente,
   dans lequel l'organe de pression (170, 174, 176) presse l'organe de support (140) de telle sorte qu'une surface de l'objet (M) soit positionnée à une position spécifiée par rapport à la source de lumière (111) et au système de photo-détection (110).

4. Appareil de formation d'image (2000) selon la revendication 3, dans lequel la position spécifiée est à l'intérieur de la fente par rapport à une direction orthogonale à la surface de l'objet (M).

5. Appareil de formation d'image (2000) selon la revendication 3 ou 4, dans lequel l'organe de support (140) a une unité de guidage configurée pour guider l'objet (M) lorsque l'objet (M) est inséré dans la fente, et

l'unité de guidage a au moins une d'une surface plane inclinée et d'une surface incurvée inclinée.

6. Appareil de formation d'image (2000) selon l'une quelconque des revendications 3 à 5, dans lequel :

   l'organe de support (140) a au moins un d'un trou débouchant et d'un organe de transmission de lumière transmettant de la lumière étant passée à travers l'objet (M) ; et
   le système de photo-détection (110) reçoit de la lumière transmise à travers au moins un du trou débouchant et de l'organe de transmission de lumière.

7. Appareil de formation d'image (2000) selon l'une quelconque des revendications 2 à 6, dans lequel l'organe de pression (170, 174, 176) presse l'objet (M) inséré dans la fente afin de ne pas limiter le mouvement relatif de l'objet (M) dans le logement (150) en commandant la force de pression.

8. Appareil de formation d'image (2000) selon l'une quelconque des revendications 1 à 7, comprenant en outre : un capteur (125) configuré pour détecter l'insertion de l'objet (M) dans la fente.

9. Appareil de formation d'image (2000) selon l'une quelconque des revendications 1 à 8, dans lequel :

   le logement (150) du dispositif capteur (100) a une paroi (119) disposée sur un côté de la fente ayant la source de lumière (111) et le système de photo-détection (110), la paroi (119) touchant l'objet (M) lorsque l'objet (M) est inséré dans la fente ; et
   la paroi (119) a une ouverture faisant passer de la lumière émise à partir de la source de lumière (111).

10. Appareil de formation d'image (2000) selon la revendication 9, dans lequel l'ouverture est formée pour avoir une direction longitudinale parallèle à une direction dans laquelle la source de lumière (111) et le système de photo-détection (110) sont disposés.

11. Appareil de formation d'image (2000) selon la revendication 9 ou 10, dans lequel l'ouverture a un bord sur un côté extrémité avant d'une direction d'insertion de l'objet (M), et le bord a un centre sur un côté extrémité avant de la direction d'insertion plus éloigné qu'au moins une de deux extrémités du bord.

12. Appareil de formation d'image (2000) selon l'une quelconque des revendications 1 à 11, dans lequel :

   la source de lumière (111) émet de la lumière linéairement polarisée ayant une première direction de polarisation ; et
   le système de photo-détection (110) comprend

   un élément optique (116) disposé sur un chemin optique de lumière réfléchie par réflexion diffuse à partir d'un plan d'incidence de l'objet (M), et configuré pour transmettre de la lumière linéairement polarisée d'une seconde direction de polarisation orthogonale à la première direction de polarisation,
   un premier photo-détecteur (113) disposé sur un chemin optique de lumière réfléchie sur l'objet (M) par réflexion régulière, et
   un deuxième photo-détecteur (114) configuré pour détecter de la lumière transmise à travers l'élément optique (116).

13. Appareil de formation d'image (2000) selon la revendication 12, dans lequel le système de photo-détection (110) comprend : un troisième photo-détecteur disposé sur un chemin optique de lumière réfléchie par réflexion diffuse à partir d'un plan d'incidence de l'objet (M).

14. Appareil de formation d'image (2000) selon une quelconque revendication précédente, dans lequel l'unité d'ajustement (130, 2090) identifie le support d'enregistrement sur la base d'une sortie provenant du dispositif capteur (100), et ajuste une condition pour la formation d'image sur la base d'un résultat d'identification.

# FIG. 1

# FIG. 2

RECORDING
PAPER

INSERTING
DIRECTION OF
RECORDING PAPER

100

SLIT

Z
Y
X

# FIG. 3

# FIG. 4A

SLIT

150

# FIG. 4B

SLIT

150

# FIG. 4C

150

SLIT

# FIG. 5

150

# FIG. 6A

140

TAPERED SURFACE
(INCLINED PLANE
SURFACE)

SUPPORTING
SURFACE

Z

Y⊗→X

# FIG. 6B

140

TAPERED SURFACE
(INCLINED PLANE
SURFACE)

SUPPORTING
SURFACE

Y

Z⊙→X

# FIG. 6C

140

INCLINED
PLANE SURFACE

SUPPORTING
SURFACE

Z

Y⊗→X

26

# FIG. 7

# FIG. 8

# FIG. 9

# FIG. 10

# FIG. 11

SURFACE EMITTING
LASER ARRAY

ELECTRODE
PAD

LIGHT EMITTING
UNIT (VCSEL)

WIRING
MEMBER

# FIG. 12

110

114

115

116

113

119

111

112

$\theta$

Z

Y

X

M

OPENING

IRRADIATION
CENTER

# FIG. 13

# FIG. 14

## FIG. 15A

SURFACE REGULAR
REFLECTION LIGHT

RECORDING
PAPER

## FIG. 15B

SURFACE DIFFUSE
REFLECTION LIGHT

RECORDING
PAPER

## FIG. 15C

INTERNAL
REFLECTION LIGHT

RECORDING
PAPER

# FIG. 16

110

114    115

Z
Y⊗──→X

111    112    116    113    119

M    IRRADIATION LIGHT    OPENING    SURFACE DIFFUSE
(S-POLARIZED LIGHT)    REFLECTION LIGHT
+ INTERNAL REFLECTION LIGHT

# FIG. 17

114

P-POLARIZED LIGHT
COMPONENT OF
INTERNAL
REFLECTION LIGHT

116

SURFACE DIFFUSE
REFLECTION LIGHT
+ INTERNAL
REFLECTION LIGHT

Z
Y⊗──→X

# FIG. 18

# FIG. 19

# FIG. 20A

110

TAPERED SURFACE
(INCLINED PLANE SURFACE)

M

INSERTING DIRECTION
OF RECORDING PAPER

140

Z
Y⊗→X

# FIG. 20B

110

M

INSERTING
DIRECTION OF
RECORDING
PAPER    TAPERED SURFACE
(INCLINED PLANE SURFACE)

140

Z
Y⊗→X

# FIG. 20C

110

M

INSERTING DIRECTION
OF RECORDING PAPER

DIRECTION OF FORCE APPLIED TO
SUPPORTING MEMBER AS
RECORDING PAPER IS INSERTED

140

Z
Y⊗→X

# FIG. 21

APPLICATION
OF FORCE

100

110

150

140

M

170  170

130

Z

Y  X

# FIG. 22

PULLING FORCE

RECORDING PAPER

INSERTING DIRECTION OF RECORDING PAPER

100

SLIT

PULLING FORCE

Y Z X

# FIG. 23

| BRAND | S1 | S2 | S3 |
|---|---|---|---|
| A | ... | ... | ... |
| B | ... | ... | ... |
| C | ... | ... | ... |
| D | ... | ... | ... |

# FIG. 24

START

↓

S401
INITIALIZE VARIABLE tc WITH 0

↓

S403
INITIALIZE VARIABLE m WITH 0

↓

S405
SWITCH ON LIGHT SOURCE

↓

S407
tc ≥ 300?

NO →

S409
OBTAIN SIGNALS OUTPUT FROM PHOTOSENSORS

↓

S411
m ← m+1

↓

S413
STORE OBTAINED DATA AND VALUE OF VARIABLE M

YES ↓

S415
SWITCH OFF LIGHT SOURCE

↓

S417
CALCULATE MEAN VALUE OF P OUTPUT LEVELS FOR EACH PHOTOSENSOR

↓

S419
CALCULATE RELEVANCE RATIO R BY REFERRING TO BRAND-SPECIFIC OUTPUT LEVEL DATA

↓

S421
IDENTIFY BRAND OF RECORDING PAPER BASED ON RELEVANCE RATIO R

↓

S423
NOTIFY PRINTER CONTROLLER OF IDENTIFIED BRAND OF RECORDING PAPER

↓

END

# FIG. 25A

100

Z → Y

X

RECORDING PAPER

# FIG. 25B

100

Z → Y

X

MOVE

RECORDING
PAPER

EP 2 848 915 B1

# FIG. 26

FIRST
DETECTION
POSITION

P-TH
DETECTION
POSITION

RECORDING PAPER

# FIG. 27

# FIG. 28

## FIG. 29

## FIG. 30

| BRAND | S1 | S2 | S3 | S4 |
|-------|-----|-----|-----|-----|
| A | ... | ... | ... | ... |
| B | ... | ... | ... | ... |
| C | ... | ... | ... | ... |
| D | ... | ... | ... | ... |

# FIG. 31

110

114

111    112

116

113    119

OPENING

M

Z
Y    X

# FIG. 32

110

114    115

120

111    112

116    113    119

121

OPENING

M

Z
Y    X

# FIG. 33

# FIG. 34

| BRAND | S1 | S2 | S3 | S5 |
|-------|-----|-----|-----|-----|
| A | ... | ... | ... | ... |
| B | ... | ... | ... | ... |
| C | ... | ... | ... | ... |
| D | ... | ... | ... | ... |

# FIG. 35

# FIG. 36A

125

TO 130

POWER SUPPLY
AND SIGNAL CABLE

125b

LIGHT

125a

# FIG. 36B

125

TO 130

POWER SUPPLY
AND SIGNAL CABLE

125b

M

LIGHT

125a

# FIG. 37

ELECTRODE
PAD

LIGHT EMITTING
UNIT (VCSEL)

WIRING
MEMBER

# FIG. 38

110

114
115

116

111  126  112
113
119

Z
Y  X

OPENING

# FIG. 39

# FIG. 40

# FIG. 41

# FIG. 42

# FIG. 43

# FIG. 44

FIG. 45

BM2

BM1

IRRADIATION
AREA

IRRADIATION
CENTER

Y

Z⊙→X

FIG. 46

K2

K1

IRRADIATION
AREA

OPENING

Y

Z⊙→X

FIG. 47

OPENING

IRRADIATION
AREA

CENTER
OF EDGE

INSERTING DIRECTION
OF RECORDING PAPER

EDGE

Y

Z⊙→X

# FIG. 48

OPENING

## FIG. 49A

OPENING

IRRADIATION AREA

CENTER OF EDGE

INSERTING DIRECTION
OF RECORDING PAPER

EDGE

Y
Z ↗ →X

## FIG. 49B

OPENING

IRRADIATION AREA

CENTER OF EDGE

INSERTING DIRECTION
OF RECORDING PAPER

EDGE

Y
Z ↗ →X

FIG. 50A

OPENING

IRRADIATION AREA

EDGE

CENTER OF EDGE

INSERTING DIRECTION OF RECORDING PAPER

EDGE

Y

Z — X

FIG. 50B

OPENING

IRRADIATION AREA

CENTER OF EDGE

INSERTING DIRECTION OF RECORDING PAPER

EDGE

Y

Z — X

# FIG. 51A

IRRADIATION AREA

INSERTING DIRECTION OF RECORDING PAPER

DIRECTION IN WHICH LIGHT SOURCE AND PHOTOSENSORS ARE ALIGNED

# FIG. 51B

OPENING

IRRADIATION AREA

INSERTING DIRECTION OF RECORDING PAPER

CENTER OF EDGE

EDGE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3730210 B
- JP 2004179721 A
- JP 2012127937 A
- US 2013057868 A1
- JP 2006062842 A
- US 2006279624 A1
- US 200906345 A1
- GB 2148194 A
- JP H03240541 A

- US 5084627 A
- US 20080063455 A1
- US 5721434 A
- US 6215552 B1
- US 5075543 A
- US 2008193157 A1
- US 2005219305 A1
- US 5915518 A